# NEW EUROPEAN PATENT SPECIFICATION

(11) **EP 0 205 564 B2**
(45) Date of publication and mention of the opposition decision: **21.06.2000**
(45) Mention of the grant of the patent: 02.05.1991
(21) Application number: 86900439.0
(22) Date of filing: 03.12.1985
(51) Int. Cl.: C12P 21/00

(54) **METHOD FOR THE PRODUCTION OF ERYTHROPOIETIN**
HERSTELLUNGSVERFAHREN FÜR ERYTHROPOIETIN
METHODE DE PRODUCTION DE L'ERYTHROPO ETINE

(30) Priority: 04.12.1984 US 677813; 03.01.1985 US 688622; 22.01.1985 US 693258
(43) Date of publication of application: 30.12.1986
(60) Divisional application: 90118215.4
(73) Proprietor: GENETICS INSTITUTE, INC., Cambridge, Massachusetts 02140 (US)
(72) Inventor: FRITSCH, Edward, Concord, MA 01742 (US); HEWICK, Rodney, M., Lexington, MA 02173 (US); JACOBS, Kenneth, Newton, MA 02160 (US)
(74) Representative: Liska, Horst, Dr.-Ing.
(86) International application number: US8502405
(87) International publication number: WO8603520

(56) References cited:
- EP-A- 0 148 605
- WO-A-85/03079
- US-A- 4 377 513
- US-A- 4 419 446
- CHEMICAL ABSTRACTS, vol. 105, no. 19, 10th November 1986, page 203, abstract no. 166280c, Columbus, Ohio, US
- Proc.Natl.Acad.Sci., USA, vol. 81, pp. 2708-2712, 1984, S. Lee Huang:"Cloning and expression of human erythropoietin cDNA in E.coli"
- Exp.Hematol, vol. 12, p. 357, 1984, F. Lin et al.:"Cloning and expression of monkey and human erythropoietin"
- Cancer, vol. 47, pp. 720-723, 1981, R. Hoffman et al.:"Erythropoiesis during an erythroblastic transformation of chronic myelocytic leukemia"
- Molec. Cell Biol., vol. 2, pp. 1304-1319, 1982, R. Kaufman et al.:"Construction of a Modular dihydrofolate reductase cDNA gene: Analysis of signals utilized for efficient expression"
- Molec. Cell Biol., vol. 3, pp. 2156-2165, 1983, G. Smith et al.:"Production of human beta interferon in insect cells infected with a baculovirus expression vector"
- Mole. Cell Biol., vol. 3, pp. 2250-2258, 1983, C. Simonsen et al.:"Analysis of processing and polyadelylation signals of the hepatitis B surface antigen gene by using SV40-hepatitis B virus chimeric plasmids"
- Science, vol. 219, pp. 620-625, 1983, R. Hitzeman et al.:"Secretion of human interferons by yeast"
- Molec. Cell Biol., vol. 3, pp. 2110-2115, 1983, M. Law et al.:"A stable papillomavirus hybrid plasmid that expresses a dominant selective trait"
- Nature, vol. 313, pp. 806-810, 1985, K. Jacobs et al.:"Isolation and characterization of genomic and cDNS clones of human erythropoietin"
- Cell, vol. 38, pp. 287-297, 1984, R. Derynck et al.:"Human transforming growth factor transforming growth factor alpha: Precursor structure and expression in E.coli"

## Description

### FIELD OF THE INVENTION

The present invention is directed to the expression of the DNA of Claim 1 and to the in vitro production of active human erythropoietin.

### BACKGROUND OF THE INVENTION

Erythropoietin (hereinafter EPO) is a circulating glycoprotein, which stimulates erythrocyte formation in higher organisms. See, Carnot et al, Compt. Rend., 143:384 (1906). As such, EPO is sometimes referred to as an erythropoiesis stimulating factor.

The life of human erythrocytes is about 120 days. Thus, about 1/120 of the total erythrocytes are destroyed daily in the reticulo-endothelial system. Concurrently, a relatively constant number of erythrocytes are produced daily to maintain the level of erythrocytes at all times (Guyton, Textbook of Medical Physiology, pp 56-60, W. B. Saunders Co., Philadelpha (1976)).

Erythrocytes are produced by the maturation and differentiation of the erythroblasts in bone marrow, and EPO is a factor which acts on less differentiated cells and induces their differentiation to erythrocytes (Guyton, supra).

EPO is a promising therapeutic agent for the clinical treatment of anemia or, in particular, renal anemia. Unfortunately, the use of EPO is not yet common in practical therapy due to its low availability.

For EPO to be used as a therapeutic agent, consideration should be given to possible antigenicity problems, and it is therefore preferable that EPO be prepared from a raw material of human origin. For example, human blood or urine from patients suffering from aplastic anemia or like diseases who excrete large amounts of EPO may be employed. These raw materials however, are in limited supply. See, for example, White et al., Rec. Progr. Horm. Res., 16:219 (1960); Espada et al., Biochem. Med., 3:475 (1970); Fisher, Pharmacol, Rev., 24:459 (1972) and Gordon, Vitam. Horm. (N.Y.) 31:105 (1973), the disclosures of which are incorporated herein by reference.

The preparation of EPO products has generally been via the concentration and purification of urine from patients exhibiting high EPO levels, such as those suffering from aplastic anemia and like diseases. See for example, U.S. Patent Nos. 4,397,840; 4,303,650 and 3,865,801 the disclosures of which are incorporated herein by reference. The limited supply of such urine is an obstacle to the practical use of EPO, and thus it is highly desirable to prepare EPO products from the urine of healthy humans. A problem in the use of urine from healthy humans is the low content of EPO therein in comparison with that from anemic patients. In addition, the urine of healthy individuals contains certain inhibiting factors which act against erthropoiesis in sufficiently high concentration so that a satisfactory therapeutic effect would be obtained from EPO derived therefrom only following significant purification.

EPO can also be recovered from sheep blood plasma, and the separation of EPO from such blood plasma has provided satisfactorily potent and stable water-soluble preparations. See, Goldwasser, Control Cellular Dif. Develop., Part A; pp 487-494, Alan R. Liss, Inc., N.Y. (1981), which is incorporated herein by reference. Sheep EPO would, however, be expected to be antigenic in humans.

Thus, while EPO is a desirable therapeutic agent, conventional isolation and purification techniques, used with natural supply sources, are inadequate for the mass production of this compound.

Sugimoto et al., in U.S. Patent No. 4,377,513 describe one method for the mass production of EPO comprising the in vivo multiplication of human lymphoblastoid cells, including Namalwa, BALL-1, NALL-1 TALL-1 and JBL.

The reported production by others of EPO using genetic engineering techniques had appeared in the trade literature. However, neither an enabling disclosure nor the chemical nature of the product has yet been published. In contrast, the present application provides an enabling disclosure for the mass production of proteins displaying the biological properties of proteins displaying the biological properties of human EPO. It is also possible by such techniques to produce proteins which may chemically differ from authentic human EPO, yet manifest similar (and in some cases improved) properties. For convenience all such proteins displaying the biological properties of human EPO may be referred to hereinafter as EPO whether or not chemically identical thereto.

### SUMMARY OF THE INVENTION

The present invention is directed to the expression of the DNA of claim 1 that expresses surprisingly high levels of human EPO, and the mass production in vitro of active human EPO therefrom. Described also are suitable expression vectors for the production of EPO, expression cells, purification schemes and related processes.

As described in greater detail infra, EPO was obtained in partially purified form and was further purified to homogeneity and digested with trypsin to generate specific fragments. These fragments were purified and sequenced. EPO oligonucleotides were designed based on these sequences and synthesized. These oligos were used to screen a human genomic library from which was isolated an EPO gene.

The EPO gene was verified on the basis of its DNA sequence which matched many of the tryptic protein fragments sequenced. A piece of the genomic clone was then used to demonstrate by hybridization that EPO mRNA could be detected in human fetal (20 weeks old) mRNA. A human fetal liver cDNA library was prepared and screened. Three EPO cDNA clones were obtained (after screening >750,000 recombinants). Two of these clones were determined to be full length as judged by complete coding sequence and substantial 5-prime and 3-prime untranslated sequence. These cDNAs have been expressed in both SV-40 virus transformed monkey cells (the COS-1 cell line; Gluzman, Cell 23:175-182 (1981)) and Chinese hamster ovary cells (the CHO cell line; Urlaub, G. and Chasin, L. A. Proc. Natl. Acad. Sci USA 77:4216-4280 (1980)). The EPO produced from COS cells is biologically active EPO in vitro and in vivo. The EPO produced from CHO cells is also biologically active in vitro and in vivo.

The EPO cDNA clone has an interesting open reading frame of 14-15 amino acids (aa) with initiator and terminator from 20 to 30 nucleotides (nt) upstream of the coding region. A representative sample of E. coli transfected with the cloned EPO gene has been deposited with the American Type Culture Collection, Rockville, Maryland, where it is available under Accession Number ATCC 40153.

### BRIEF DESCRIPTION OF DRAWINGS AND TABLES

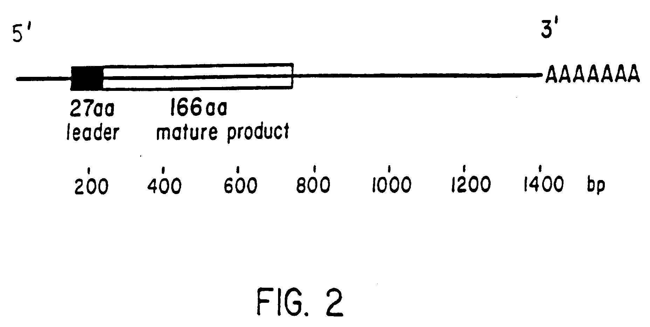

Table 1 is the base sequence of an 87 base pair exon of a human EPO gene;
Figure 1 illustrates the detection of EPO mRNA in human fetal liver mRNA;
Table 2 illustrates the amino acid sequence of an EPO protein deduced from the nucleotide sequence of lambda-HEPOFL13.;
Table 3 illustrates the nucleotide sequence of the EPO cDNA in lambda-HEPOFL13 (shown schematically in Figure 2) and the amino acid sequence deduced therefrom;
Figure 3 illustrates the relative positions of DNA inserts of four independent human EPO genomic clones;
Figure 4 illustrates a map of the apparent intron and exon structure of the human EPO gene;
Table 4 illustrates a DNA sequence of the EPO gene;
Figures 5A, 5B and 5C illustrate the construction of the vector 91023(B);
Figure 6 illustrates SDS polyacrylamide gel analysis of EPO produced in COS-1 cells compared with native EPO;
Table 5 illustrates the nucleotide and amino acid sequence of the EPO clone, lambda-HEPOFL6;
Table 6 illustrates the nucleotide and amino acid sequence of the EPO clone, lambda-HEPOFL8;
Table 7 illustrates the nucleotide and amino acid sequence of the EPO clone lambda-HEPOFL13;
Figure 7 is a schematic illustration of the plasmid pRK1-4; and
Figure 8 is a schematic illustration of the plasmid pdBPV-MMTneo(342-12).

### DETAILED DESCRIPTION

The present invention is directed to the production of EPO by the in vitro expression of the DNA of claim 1.

The patent and scientific literature is replete with processes reportedly useful for the production of recombinant products. Generally, these techniques involve the isolation or synthesis of a desired gene sequence, and the expression of that sequence in either a procaryotic or eucaryotic cell, using techniques commonly available to the skilled artisan. Once a given gene has been isolated, purified and inserted into a transfer vector (i.e., cloned), its availability in substantial quantity is assured. The vector with its cloned gene is transferred to a suitable microorganism or cell line, for example, bacteria, yeast, mammalian cells such as, COS-1 (monkey kidney), CHO (Chinese hamster ovary), insect cell lines, and the like, wherein the vector replicates as the microorganism or cell line proliferates and from which the vector can be isolated by conventional means. Thus there is provided a continuously renewable source of the gene for further manipulations, modifications and transfers to other vectors or other loci within the same vector.

Expression may often be obtained by transferring the cloned gene, in proper orientation and reading frame, into an appropriate site in a transfer vector such that translational read-through from a procaryotic or eucaryotic gene results in synthesis of a protein precursor comprising the amino acid sequence coded by the cloned gene linked to Met or an amino-terminal sequence from the procaryotic or eucaryotic gene. In other cases, the signals for transcription and translation initiation can be supplied by a suitable genomic fragment of the cloned gene. A variety of specific protein cleavage techniques may be used to cleave the protein precursor, if produced, at a desired point so as to release the desired amino acid sequence, which may then be purified by conventional means. In some cases, the protein containing the desired amino acid sequence is produced without the need for specific cleavage techniques and may also be released from the cells into the extracellular growth medium.

### Isolation of a Genomic Clone of Human EPO

Human EPO was purified to homogeneity from the urine of patients afflicted with aplastic anemia as described infra. Complete digestion of this purified EPO with the protease trypsin, yielded fragments which were separated by reverse phase high performance liquid chromatography, recovered from gradient fractions, and subjected to microsequence analysis. The sequences of the tryptic fragments are underlined in Tables 2 and 3 and are discussed in more detail infra. Two of the amino acid sequences, Val-Asn-Phe-TyrAla-Trp-Lys and Val-Tyr-Ser-Asn-Phe-Leu-Arg, were chosen for the design of oligonucleotide probes (resulting in an oligonucleotide pool 17nt long and 32-fold degenerate, and an oligonucleotide pool 18nt long and 128-fold degenerate, from the former tryptic fragment, as well as two pools 14nt long, each 48-fold degenerate, from the latter tryptic fragment, respectively). The 32-fold degenerate 17mer pool was used to screen a human genomic DNA library in a Ch4A vector (22) using a modification of the Woo and O'Malley in situ amplification procedure (47) to prepare the filters for screening.

As used herein, arabic numbers in parentheses, (1) through (61), are used to refer to publications that are listed in numerical order at the end of this specification.

Phage hybridizing to the 17mer were picked, pooled in small groups and probed with the 14mer and 18mer pools. Phage hybridizing to the 17mer, 18mer and 14mer pools were plaque purified and fragments were subcloned into M13 vectors for sequencing by the dideoxy chain termination method of

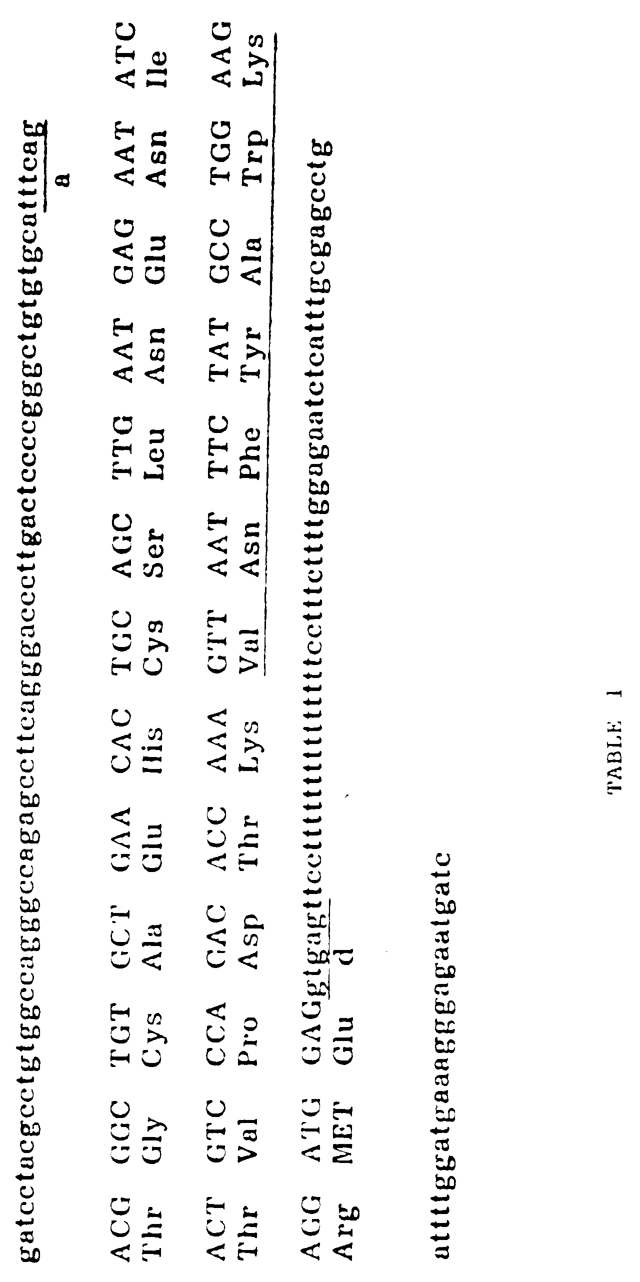

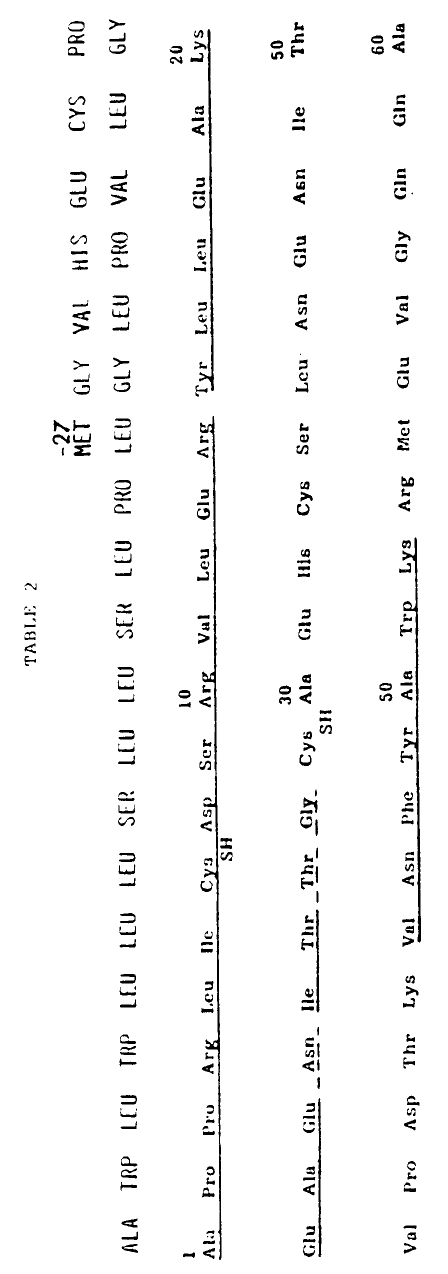

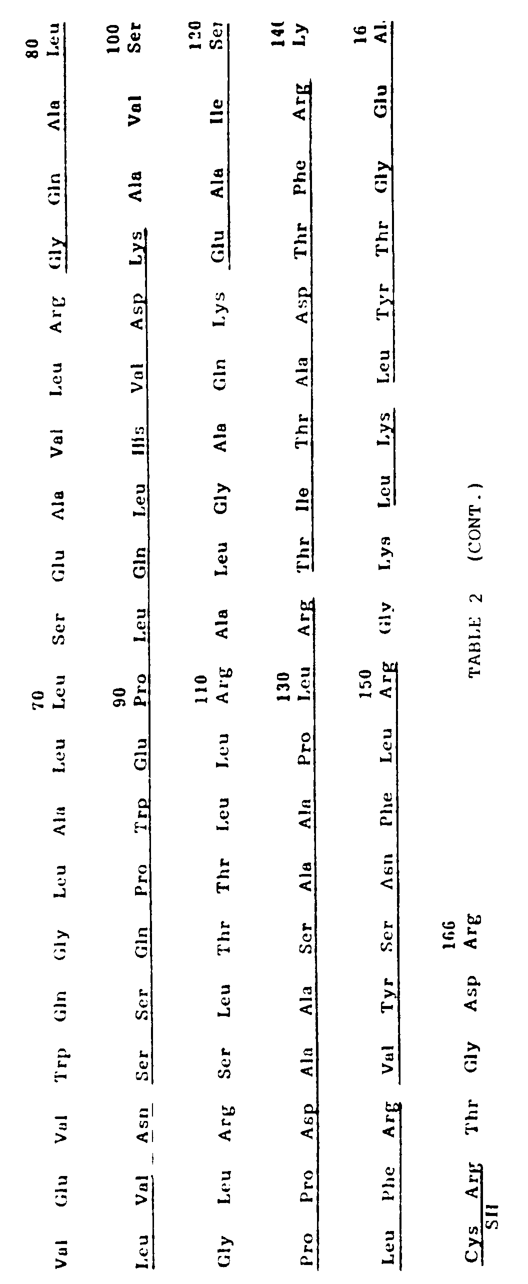

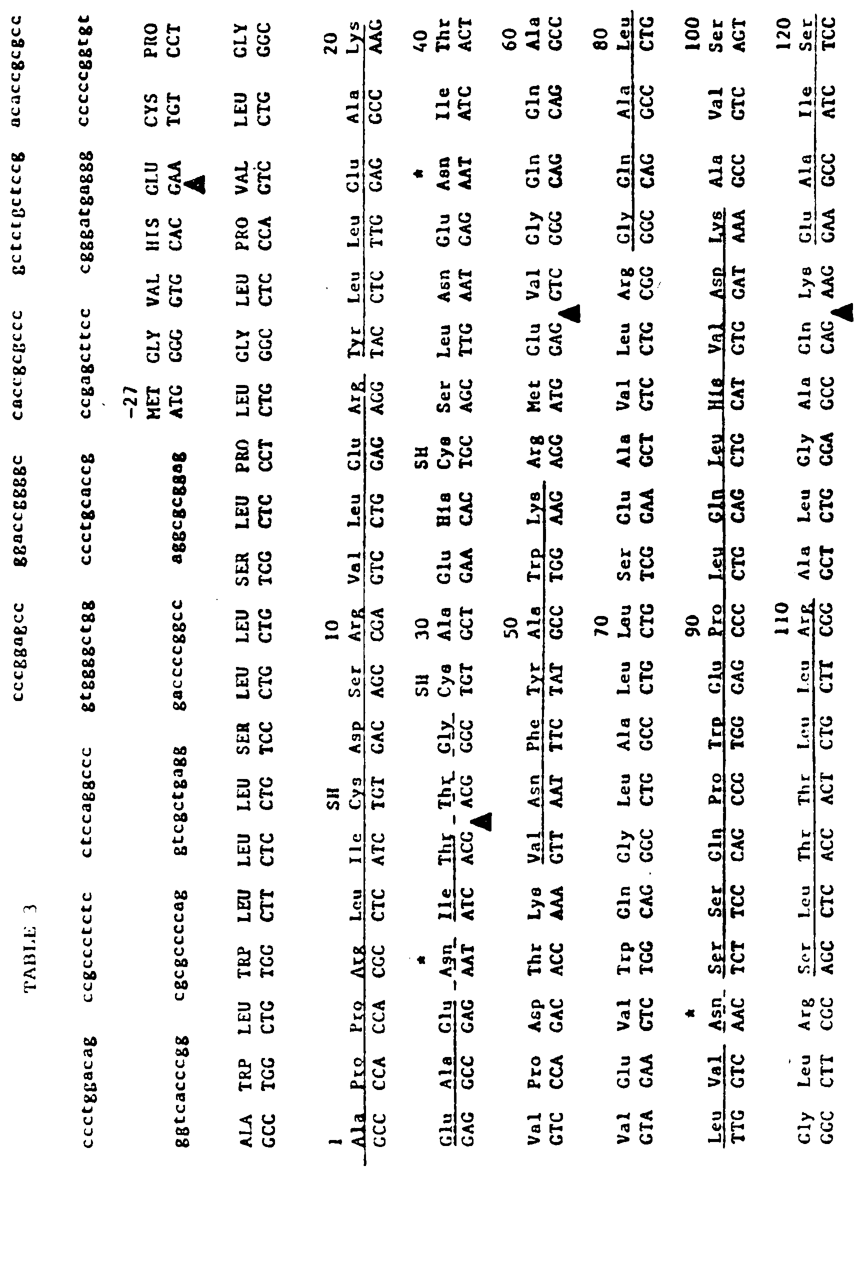

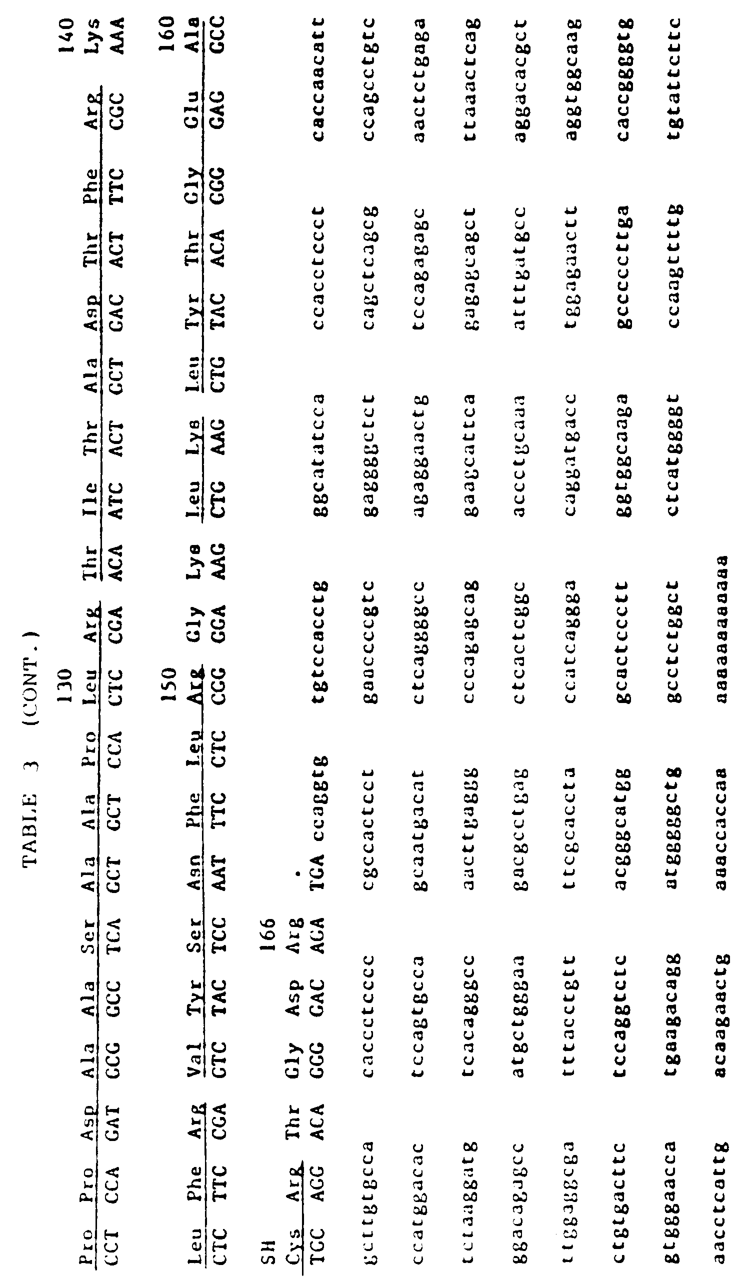

Sanger and Coulson, (23) (1977). The sequence of the region hybridizing to the 32-fold degenerate 17mer in one of the clones is shown in Table 1. This DNA sequence contains within an open reading frame, the nucleotides which could precisely code for the tryptic fragment used to deduce the 17mer pool of oligonucleotides. Furthermore, analysis of the DNA sequence indicated that the 17mer hybridizing region was contained within an 87bp exon, bounded by potential splice acceptor and donor sites.

Positive confirmation that these two clones (designated herein, lambda-HEPO1 and lambda-HEPO2) are EPO genomic clones has been obtained by sequencing additional exons containing other tryptic fragment coding information.

### Isolation of EPO cDNA Clones

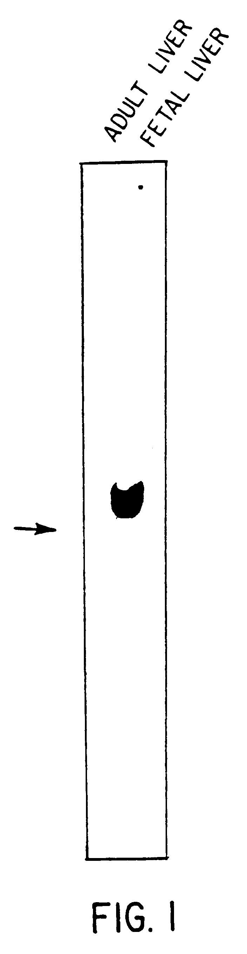

Northern Analysis (56) of human fetal (20 weeks old) liver mRNA was conducted using a 95nt single-stranded probe prepared from an M13 clone containing a portion of the 87bp exon described in Table 1. As illustrated in Figure 1, a strong signal could be detected in fetal liver mRNA. The precise identification of this band as EPO mRNA was achieved by using the same probe to screen a bacteriophage lambda cDNA library of the fetal liver mRNA (25). Several hybridizing clones were obtained at a frequency of approximately 1 positive per 250,000 recombinants screened. The complete nucleotide and deduced amino acid sequences for these clones (lambda-HEPOFL13 and lambda-HEPOFL8) are shown in Tables 7 and 6. The EPO coding information is contained within 594nt in the 5-prime half of the cDNA, including a very hydrophobic 27 amino acid leader and the 166 amino acid mature protein.

The identification of the N-terminus of the mature protein was based on the N-terminal sequence of the protein secreted in the urine of persons with aplastic anemia as illustrated herein (Table 1), and as published by Goldwasser (26), Sue and Sytkowski (27), and by Yangawa (21). Whether this N-terminus (Ala-Pro-Pro-Arg---) represents the actual N-terminus found on EPO in circulation or whether some cleavage occurs in the kidney or urine is presently unknown.

The amino acid sequences which are underlined in Tables 2 and 3 indicate those tryptic fragments or the portion of the N-terminus for which protein sequence information was obtained. The deduced amino acid sequence agrees precisely with the tryptic fragments which have been sequenced, confirming that the isolated gene encodes human EPO.

### Structure and Sequence of the Human EPO Gene

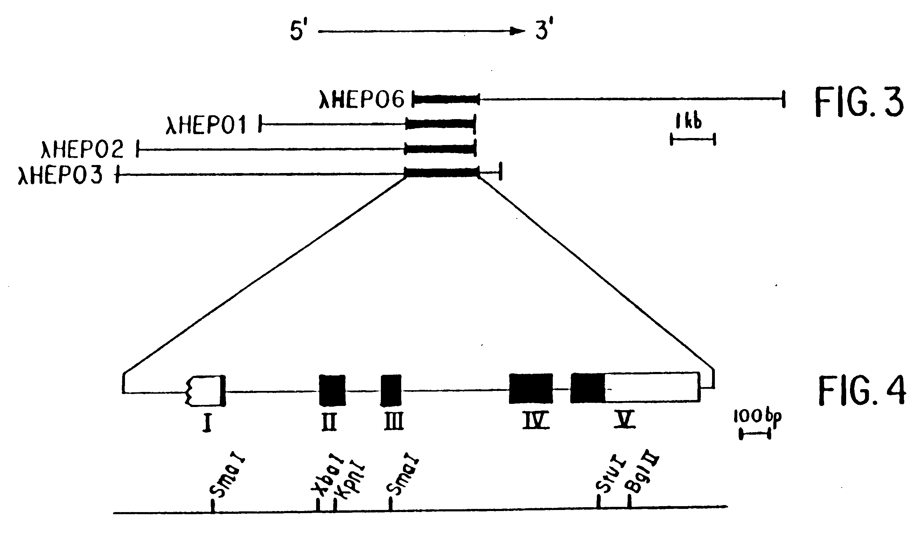

The relative positions of the DNA inserts of four independent human EPO genomic clones are shown in Figure 3. Hybridization analysis of these cloned DNAs with oligonucleotide probes and with various probes prepared from the two classes of EPO cDNA clones positioned the EPO gene within the approximately 3.3 kb region shown by the darkened line in Figure 3. Complete sequence analysis of this region (see Example 4) and comparison with the cDNA clones, resulted in the map of the intron and exon structure of the EPO gene shown in Figure 4. The EPO gene is divided into 5 exons. Part of exon I, all of exons II, III and IV, and part of exon V, contain the protein coding information. The remainder of exons I and V encode the 5-prime and the 3-prime untranslated sequences respectively.

### Transient Expression of EPO in COS Cells

To demonstrate that biologically active EPO could be expressed in an in vitro cell culture system, COS cell expression studies were conducted (58). The vector used for the transient studies, p91023(B), is described in Example 5. This vector contains the adenovirus major late promoter, an SV40 polyadenylation sequence, an SV40 origin of replication, SV40 enhancer, and the adenovirus VA gene. The cDNA insert in lambda-HEPOFL13 (see Table 6) was inserted into the p91023(B) vector, downstream of the adenovirus major late promoter. This new vector is identified as pPTFL13.

Twenty four hours after transfection of this construct into the M6 strain of COS-1 cells (Horowitz et al, J. Mol. Appl. Genet. 2:147-149 (1983)), the cells were washed, changed to serum free media, and the cells were harvested 48 hrs. later. The level of release of EPO into the culture supernatant was then examined using a quantitative radioimmunoassay for EPO (55). As shown in Table 8, (Example 6) immunologically reactive EPO was expressed. The biological activity of the EPO produced from COS-1 cells was also examined. In a separate experiment, the vector containing EPO cDNA from lambda-HEPOFL13 was transfected into COS-1 cells and media harvested as described supra. EPO in the media was then quantified by the either of two in vitro biological assays, ³H-thymidine and CFU-E (12, 29), and by either of two in vivo assays, hypoxic mouse and starved rat (30, 31) (see Table 9, Example 7). These results demonstrate that biologically active EPO is produced in COS-1 cells. By Western blotting, using a polyclonal anti-EPO antibody, the EPO produced by COS cells has a mobility on SDS-polyacrylamide gels which is identical to that of native EPO prepared from human urine (Example 8). Thus, the extent of glycosylation of COS-1 produced EPO may be similar to that of native EPO.

Different vectors containing other promoters can also be used in COS cells or in other mammalian or eukaryotic cells. Examples of such other promoters useful in the practice of this invention include SV40 early and late promoters, the mouse metallothionein gene promoter, the promoter found in the long terminal repeats of avian or mammalian retroviruses, the bacculovirus polyhedron gene promoter and others. Examples of other cell types useful in the practice of this invention include E. coli, yeast, mammalian cells such as CHO (Chinese hamster ovary), C127 (monkey epithelium), 3T3 (mouse fibroblast) CV-1 (African green monkey kidney), and the insect cells such as those from Spodoptera frugiperda and Drosophila melanogaster. These alternate promoters and/or cell types may enable regulation of the timing or level of EPO expression, producing a cell-specific type of EPO, or the growth of large quantities of EPO producing cells under less expensive, more easily controlled conditions.

An expression system which retains the benefits of mammalian expression but requires less time to produce a high-level expression cell line is composed of an insect cell line and a DNA virus which reproduces in this cell line. The virus is a nuclear polyhedrosis virus. It has a double-stranded circular DNA genome of 128 kb. The nucleocapsid is rod-shaped and found packaged in two forms, the non-occluded form, a membrane budded virus and an occluded form, packaged in a protein crystal in the infected cell nucleus. These viruses can be routinely propagated in in vitro insect cell culture and are amendable to all routine animal virological methods. The cell culture media is typically a nutrient salt solution and 10% fetal calf serum.

In vitro, virus growth is initiated when a non-occluded virus (NOV) enters a cell and moves to the nucleus where it replicates. Replication is nuclear. During the initial phase (8-18 hrs. post-infection) of viral application, nucleocapsids are assembled in the nucleus and subsequently BUD through the plasma membrane as NOVs, spreading the infection through the cell culture. In addition, some of the nucleocapsids subsequently (18+ hrs. post-infection) remain in the nucleus and are occluded in a protein matrix, known as the polyhedral inclusion body (PIB). This form is not infectious in cell culture. The matrix is composed of a protein known as polyhedrin, MW 33 kd. Each PIB is approximately 1 mm in diameter, and there can be as many as 100 PIBs per nucleus. There is clearly a great deal of polyhedrin produced late in the infection cycle, as much as 25% of total cellular protein.

Because the PIB plays no role in the in vitro replication cycle, the polyhedrin gene can be deleted from the virus chromosome with no effect on in vitro viability. In using the virus as an expression vector, we have replaced the polyhedrin gene coding region with the foreign DNA to be expressed, placing it under the control of the polyhedrin promoter. This results in a non-PIB forming virus phenotype.

This system has been utilized by several researchers the most noted being Pennock et al. and Smith et al. Pennock et al. (Gregory D. Pennock, Charles Shoemaker, and Lois K. Miller, Molecular and Cell Biology 3:84. p. 399-406) have reported on the high level expression of a bacterial protein, β-galactosidase, when placed under the control of the polyhedrin promoter.

Another nuclear polyhedrosis virus-derived expression vector has been presented by Smith et al. (Gale E. Smith, Max D. Summers and M. J. Fraser, Molecular and Cell Biology, May 16, 1983, pp. 2156-2165). They have demonstrated the effectiveness of their vector through the expression of human β-interferon. The synthesized product was found to be glycosylated and secreted from insect cells, as would be expected. In Example 14, modifications to the plasmid containing the Autographa californica nuclear polyhedrosis virus (AcNPV) polyhedron gene are described which allow the easy insertion of the EPO gene into the plasmid so that it may be under the transcriptional control of the polyhedrin promoter. The resulting DNA is co-transfected with intact chromosome DNA from wild type AcNPV into insect cells. A genetic recombination event results in the replacement of the AcNPVC polyhedrin gene region with the DNA from the plasmid. The resulting recombinant virus can be identified amongst the viral progeny by its possession of the DNA sequences of the EPO gene. This recombinant virus, upon reinfection of insect cells is expected to produce EPO.

Examples of EPO expression in CHO, C127 and 3T3, and insect cells are given in Examples 10 and 11 (CHO), 13 (C127 and 3T3) and 14 (insect cells).

Recombinant EPO produced in CHO cells as in Example 11 was purified by conventional column chromatographic methods. The relative amounts of sugars present in the glycoprotein were analyzed by two independent methods [(i)Reinhold, Methods in Enzymol. 50:244-249 (Methanolysis) and (ii) Takemoto, H. et al., Anal. Biochem. 145:245 (1985) (pyridyl amination, together with independent sialic acid determination)]. The results obtained by each of these methods were in excellent agreement. Several determinations were thus made, yielding the following average values wherein N-acetylglucosamine is, for comparative purposes, given a value of 1:

| Sugar | Relative molar level |
|---|---|
| N-Acetylglucosamine | 1 |
| Hexoses: | 1.4 |
| Galactose | 0.9 |
| Mannose | 0.5 |
| N-Acetylneuraminic acid | 1 |
| Fucose | 0.2 |
| N-Acetylgalactosamine | 0.1 |

It is noteworthy that significant levels of fucose and N-acetylgalactosamine were reproducibly observed using both independent methods of sugar analysis. The presence of N-acetylgalactosamine indicates the presence of O-linked glycosylation on the protein. The presence of O-linked glycosylation was further indicated by SDS-PAGE analysis of the glycoprotein following digestion of the glycoprotein with various combinations of glycosidic enzymes. In particular, following enzymatic removal of all N-linked carbohydrate on the glycoproteins using the enzyme peptide endo F N-glycosidase, the molecular weight of the protein was further reduced upon subsequent digestion with neuraminidase, as determined by SDS-PAGE analysis.

In vitro biological activity of the purified recombinant EPO was assayed by the method of G. Krystal, Exp. Hematol. 11:649 (1983) (spleen cell proliferation bioassay) with protein determinations calculated based upon amino acid compositional data. Upon multiple determinations, the in vitro specific activity of the purified recombinant EPO was calculated to be greater than 200,000 units/mg protein. The average value was in the range of about 275,000 - 300,000 units/mg. protein. Moreover, values higher than 300,000 have also been observed. The in vivo (polycythemic mouse assay, Kazal and Erslev, Am. Clinical Lab. Sci., Vol. B, p. 91 (1975))/in vitro activity ratios observed for the recombinant material was in the range of 0.7 - 1.3.

It is interesting to compare the glycoprotein characterization presented above with the characterization for a recombinant CHO-produced EPO material previously reported in International Patent Application Publication No. WO 85/02610 (published 20 June 1985). The corresponding comparative sugar analysis described on page 65 of that application reported a value of zero for fucose and for N-acetylgalactosamine and a hexoses:N-acetylgalactosamine ratio of 15.09:1. The absence of N-acetylgalactosamine indicates the absence of O-linked glycosylation in the previously reported glycoprotein. In contrast to that material, the recombinant CHO-produced EPO of this invention which is characterized above contains significant and reproducibly observable amounts of both fucose and N-acetylgalactosamine, contains less than one-tenth the relative amount of hexoses and is characterized by the presence of O-linked glycosylation. Furthermore, the high specific activity of the above-described CHO-derived recombinant EPO of this invention may be directly related to its characteristic glycosylation pattern.

The biologically active EPO produced by the eucaryotic expression of the cloned EPO-DNA of claim 1 of the present invention can be used for the in vivo treatment of mammalian species by physicians and/or veterinarians. The amount of active ingredient will, of course, depend upon the severity of the condition being treated, the route of administration chosen, and the specific activity of the active EPO, and ultimately will be decided by the attending physician or veterinarian. Such amount of active EPO was determined by the attending physician is also referred to herein as an "EPO treatment effective" amount. For example, in the treatment of induced hypoproliferative anemia associated with chronic renal failure in sheep, an effective daily amount of EPO was found to be 10 units/kg for from 15 to 40 days. See Eschbach et al., J. Clin. Invest., 74:434 (1984).

The active EPO may be administered by any route appropriate to the condition being treated. Preferably, the EPO is injected into the bloodstream of the mammal being treated. It will be readily appreciated by those skilled in the art that the preferred route will vary with the condition being treated.

While it is possible for the active EPO to be administered as the pure or substantially pure compound, it is preferable to present it as a pharmaceutical formulation or preparation.

The formulations, both for veterinary and for human use, comprise an active EPO protein, as above described, together with one or more pharmaceutically acceptable carriers therefor and optionally other therapeutic ingredients. The carrier(s) must be "acceptable" in the sense of being compatible with the other ingredients of the formulation and not deleterious to the recipient thereof. Desirably the formulation should not include oxidizing agents and other substances with which peptides are known to be incompatible. The formulations may conveniently be presented in unit dosage form and may be prepared by any of the methods well known in the art of pharmacy. All methods include the step of bringing into association the active ingredient with the carrier which constitutes one or more accessory ingredients. In general, the formulations are prepared by uniformly and intimately bringing into association the active ingredient with liquid carriers or finely divided solid carriers or both, and then, if necessary, shaping the product into the desired formulation.

Formulations suitable for parenteral administration conveniently comprise sterile aqueous solutions of the active ingredient with solutions which are preferably isotonic with the blood of the recipient. Such formulations may be conveniently prepared by dissolving solid active ingredient in water to produce an aqueous solution, and rendering said solution sterile may be presented in unit or multi-dose containers, for example sealed ampoules or vials.

EPO/cDNA as used herein includes the mature EPO/cDNA gene preceded by an ATG codon and EPO/cDNA coding for EPO protein as shown in Tables 2 and 3. The EPO protein includes the 1-methionine derivative of EPO protein (Met-EPO). The mature EPO protein illustrated by the sequence in Table 2 begins with the sequence Ala.Pro.Pro.Arg...the beginning of which is depicted by the number "1" in Table 2. The Met-EPO would begin with the sequence Met.Ala.Pro.Pro.Arg...

The following examples are provided to aid in the understanding of the present invention, the true scope of which is set forth in the appended claims. All temperatures are expressed in degrees Celsius and are uncorrected. The symbol for micron or micro, e. g., microliter, micromole, etc., is "u", e.g., ul, um, etc.

### EXAMPLES

### Example I: Isolation of a Genomic Clone of EPO

EPO was purified from the urine of patients with aplastic anemia essentially as described previously (Miyake, et al., J. Biol. Chem., 252:5558 (1977)) except that the phenol treatment was eliminated and replaced by heat treatment at 80 deg. for 5 min. to inactivate neuraminidase. The final step in the purification was fractionation on a C-4 Vydac HPLC column (The Separations Group) using 0 to 95% acetonitrile gradient with 0.1% trifluoracetic acid (TFA) over 100 minutes. The position of EPO in the gradient was determined by gel electrophoresis and N-terminal sequence analysis (21, 26, 27) of the major peaks. The EPO was eluted at approximately 53% acetonitrile and represented approximately 40% of the protein subjected to reverse phase - HPLC. Fractions containing EPO were evaporated to 100 ul, adjusted to pH 7.0 with ammonium bicarbonate digested to completion with 2% TPCK-treated trypsin (Worthington) for 18 hrs. at 37 deg. The trypic digestion was then subjected to reverse phase HPLC as described above. The optical density at both 280 and 214 nm was monitored. Well separated peaks were evaporated to near dryness, and subjected directly to N-terminal amino acid sequence analysis (59) using an Applied Biosystems Model 480A gas phase sequenator. The sequences obtained are underlined in Tables 2 and 3. As described herein supra, two of these tryptic fragments were chosen for synthesis of oligonucleotide probes. From the sequence, Val-Asn-Phe-Tyr-Ala-Trp-Lys (amino acids 46 through 52 in Tables 2 and 3), a 17mer of 32 fold degeneracy
TTCCANGCGTAGAAGTT
and an 18mer of 128 fold degeneracy
CCANGCGTAGAAGTTNAC
were prepared. From the sequence, Val-Tyr-Ser-Asn-Phe-Leu-Arg (amino acids 144 through 150 in Tables 2 and 3), two pools of 14mers,
each 32-fold degenerate
TACACCTAACTTCCT and TACACCTAACTTCTT
which differ at the first position of the leucine codon were prepared. The oligonucleotides were labelled at the 5-prime end with ³²P using polynucleotide kinase (New England Biolabs) and gamma ³²P-ATP (New England Nuclear). The specific activity of the oligonucleotides varied between 1000 and 3000 Ci/mmole oligonucleotide. A human genomic DNA library in bacteriophage lambda (Lawn et al., 22) was screened using a modification of the in situ amplification procedure originally described by Woo et al., (47) (1978). Approximately 3.5 x 10⁵ phages were plated at a density of 6000 phages per 150 mm petri dish (NZCYM media) and incubated at 37 deg. until the plaques were visible, but small (approximately 0.5 mm). After chilling at 4 deg. for 1 hr., duplicate replicas of the plaque patterns were transferred to nylon membranes (New England Nuclear) and incubated overnight at 37 deg. on fresh NZCYM plates. The filters were then denatured and neutralized by floating for 10 min. each on a thin film of 0.5N NaOH - 1M NaCl and 0.5M Tris (pH 8) -1M NaCl respectively. Following vacuum baking at 80 deg. for 2 hrs., the filters were washed in 5 x SSC, 0.5% SDS for 1 hr. and the cellular debris on the filter surface was removed by gentle scrapping with a wet tissue. This scrapping reduced the background binding of the probe to the filters. The filters were then rinsed with H₂O and prehybridized for from 4 to 8 hrs. at 48 deg. in 3M tetramethylammonium chloride, 10 mM NaPO₄ (pH 6.8), 5 x Denhardt's, 0.5% SDS and 10mM EDTA. The ³²P-labeled 17mer was then added at a concentration of 0.1 pmol/ml and hybridization was carried out at 48 deg. for 72 hrs. Following hybridization the filters were washed extensively in 2 x SSC (0.3M NaCl - 0.03M Na citrate, pH 7) at room temperature and then for 1 hr. in 3M TMACl - 10mM NaPO₄ (pH 6.8) at room temperature and from 5 to 15 min. at the hybridization temperature. Approximately 120 strong duplicate signals were detected following 2 day autoradiography with an intensifying screen. The positives were picked, grouped in pools of 8, replated and rescreened in triplicate using one-half of the 14mer pool on each of two filters and the 127mer on the third filter. The conditions and the 17mer for plating and hybridization were as described supra except that hybridization for the 14mer was at 37 deg. Following autoradiography, the probe was removed from the 17mer filter in 50% formamide for 20 min. at room temperature and the filter was rehybridized at 52 deg. with the 18mer probe. Two independent phage hybridized to all three probes. DNA from one of these phage (designated herein, lambda HEPO1) was digested to completion with Sau3A and subcloned into M13 for DNA sequence analysis using the dideoxy chain termination method of Sanger and Coulson, (23) (1977). The nucleotide sequence and deduced amino acid sequence of the open reading frame coding for the EPO tryptic fragment (underlined region) are described herein. Intron sequences are given in lower case letters; exon sequences (87nt) are given in upper case. Sequences which agree with consensus splice acceptor (a) and donor (d) sites are underlined. (See Table 4.)

### Example 2: Northern Analysis of Human Fetal Liver mRNA

5 ug of human fetal liver mRNA (prepared from a 20 weeks old fetal liver) and adult liver mRNA were electro

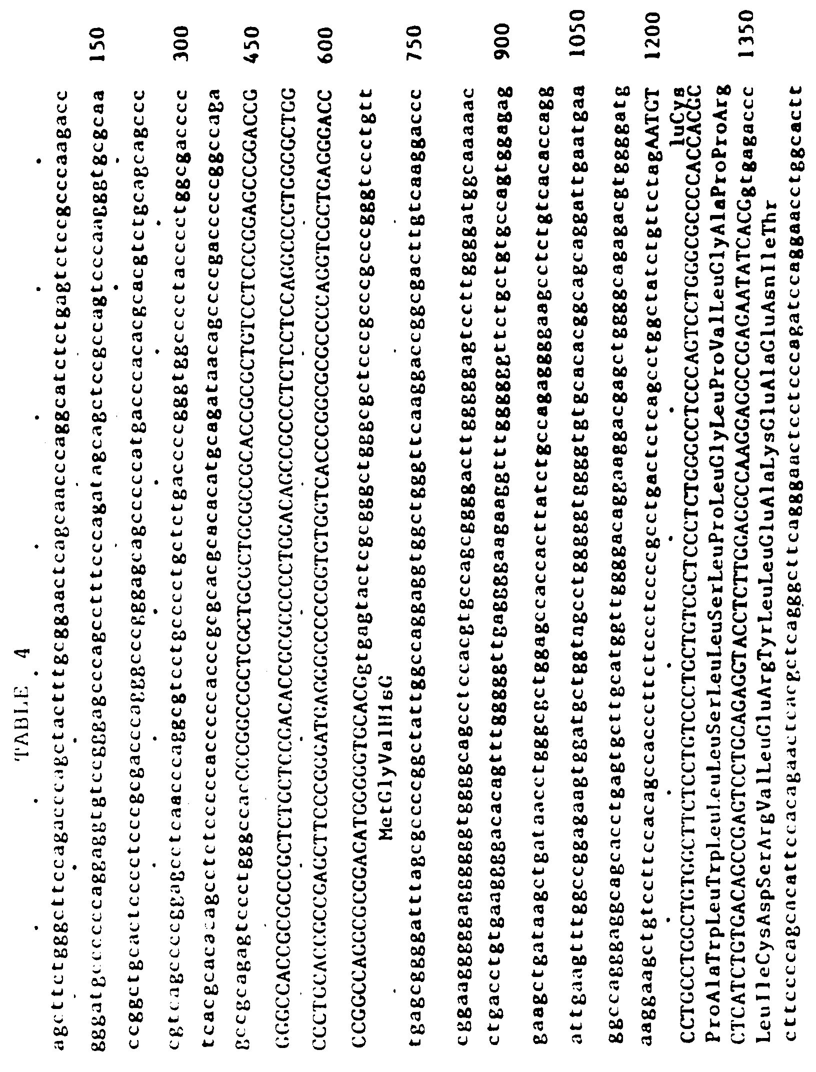

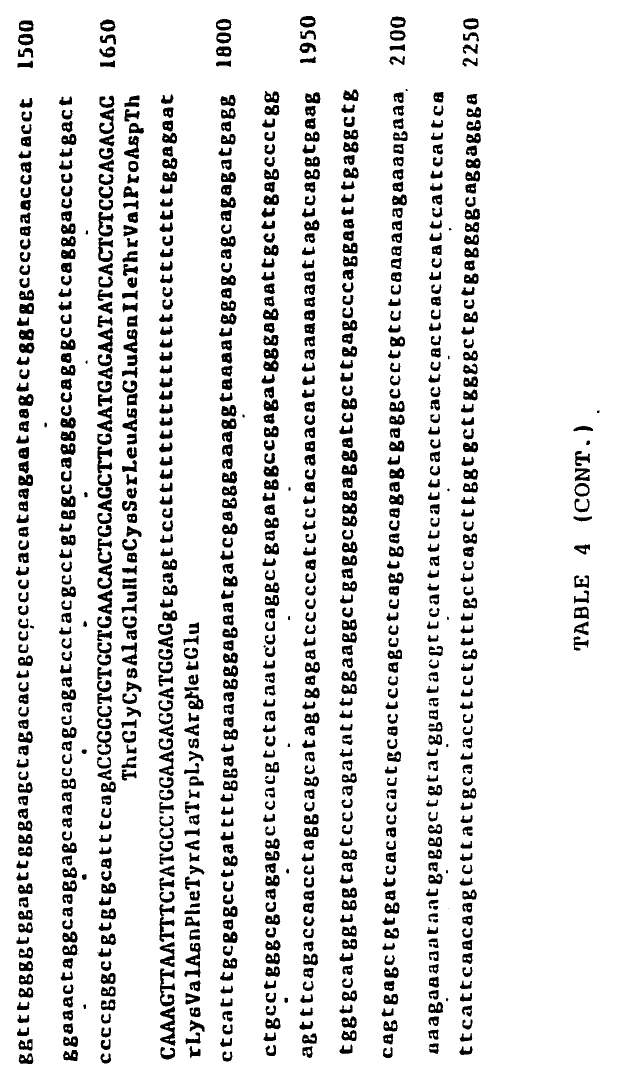

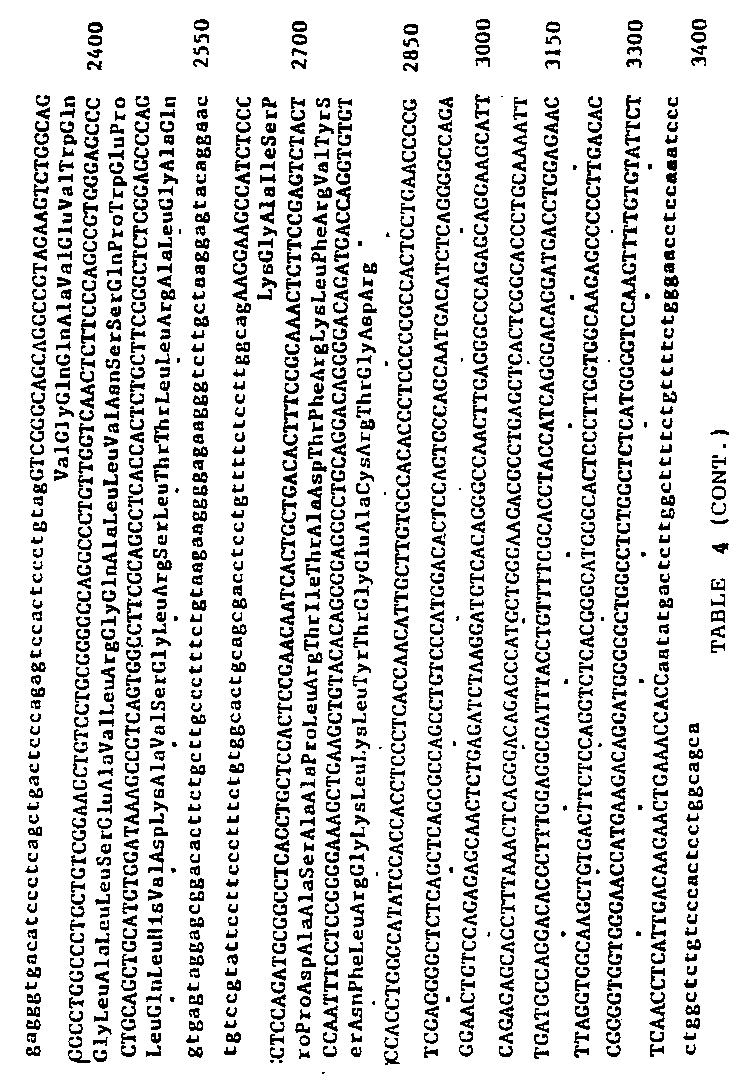

phoresed in a 0.8% agarose formaldehyde gel and transferred to nitrocellulose using the method of Derman et al., Cell, 23:731 (1981). A single-stranded probe was then prepared from an M13 template containing the insert illustrated in Table 1. The primer was a 20mer derived from the same tryptic fragment as the original 17mer probe. The probe was prepared as previously described by Anderson et al., PNAS, (50) (1984) except that, following digestion with SmaI (which produced the desired probe of 95nt length containing 74nt of coding sequence), the small fragment was purified from the M13 template by chromatography on a sepharose C14B column in 0.1N NaOH - 0.2M NaCl. The filter was hybridized to approximately 5 x 10⁶ cpm of this probe for 12 hrs. at 68 deg., washed in 2 x SSC at 68 deg. and exposed for 6 days with an intensifying screen. A single marker mRNA of 1200 nt (indicated by the arrow) was run in an adjacent lane. (Figure 1).

### Example 3: Fetal Liver cDNA

A probe identical to that described in Example 2 was prepared and used to screen a fetal liver cDNA library prepared in the vector lambda-Ch21A (Toole et al., Nature, (25) (1984)) using standard plaque screening (Benton Davis, Science, (54) (1978)) procedures. Three independent positive clones (designated herein, lambda-HEPOFL6 (1350bp), lambda-HEPOFL8 (700bp) and lambda-HEPOFL13 (1400bp) were isolated following screening of 1 x 10⁶ plaques. The entire inserts of lambda-HEPOFL13 and lambda-HEPOFL6 were sequenced following subcloning into M13. (Tables 7 and 5, respectively). Only portions of lambda-HEPOFL8 were sequenced and the remainder assumed to be identical to the other two clones. (Table 6). The 5-prime and 3-prime untranslated sequences are represented by lower case letters. The coding region is represented by upper case letters.

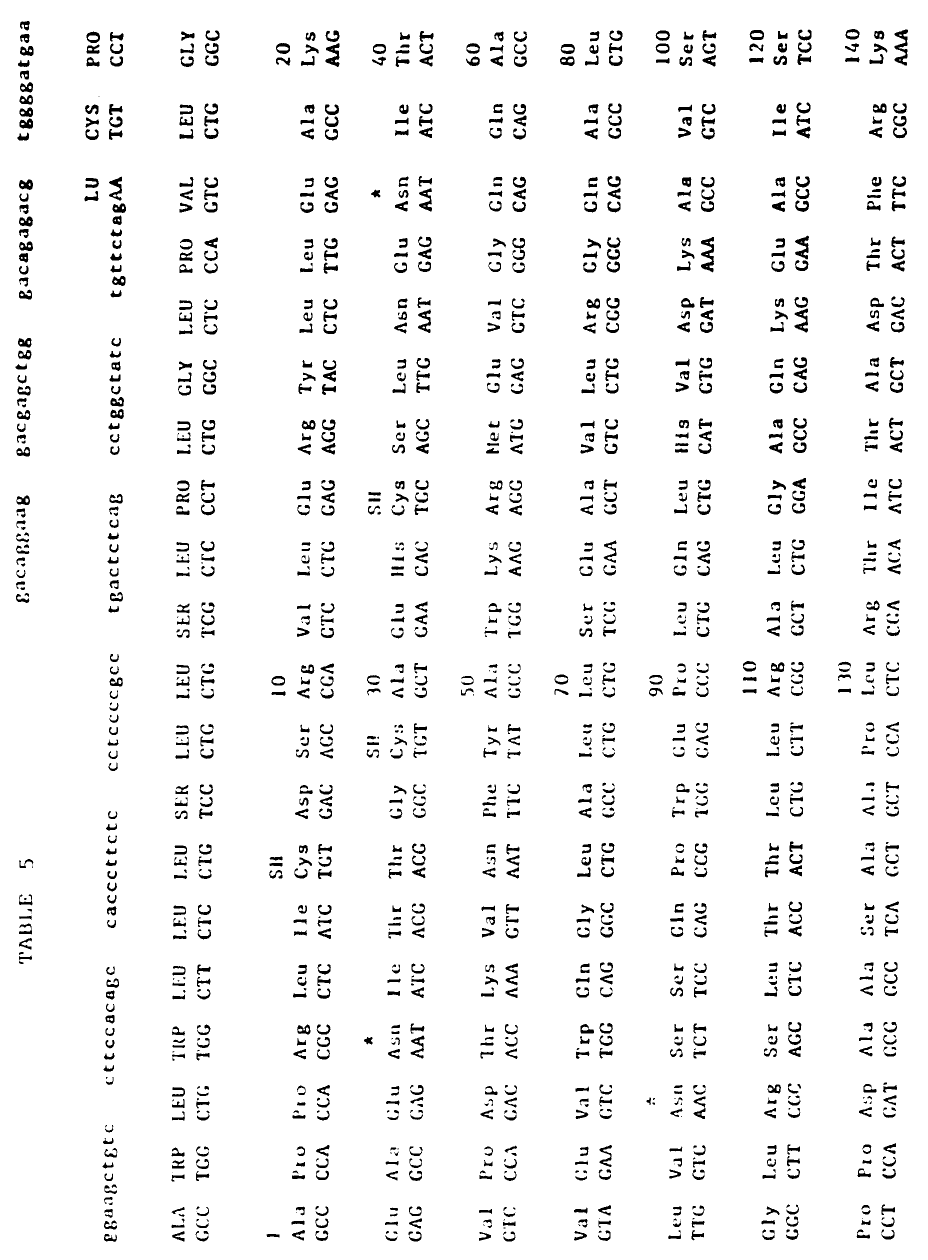

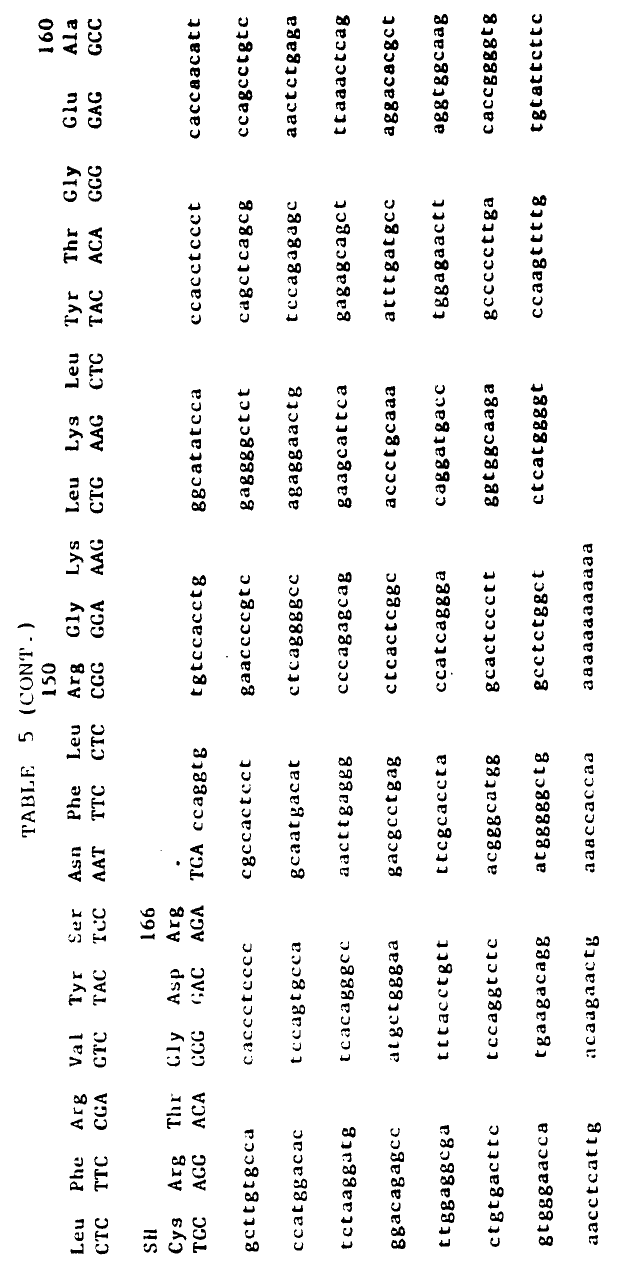

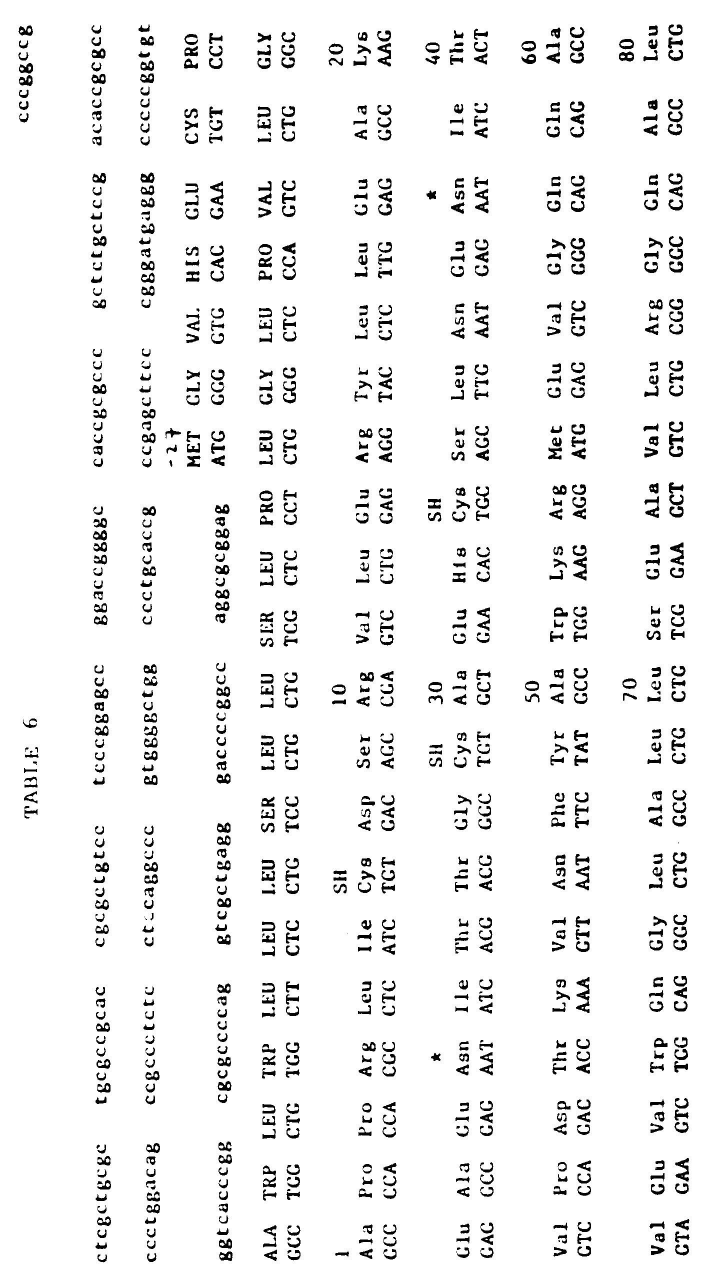

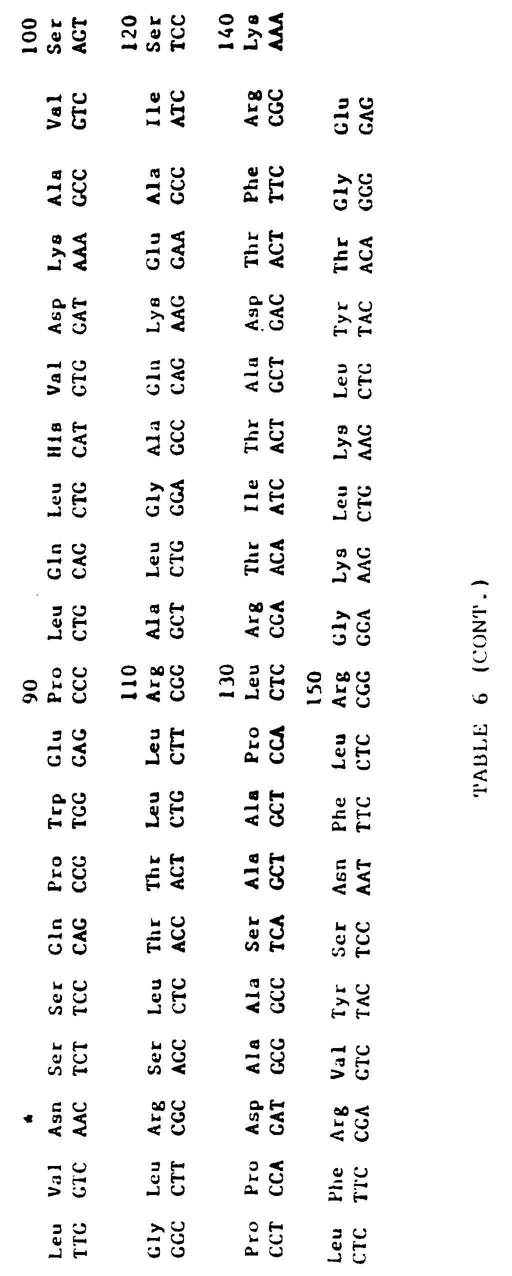

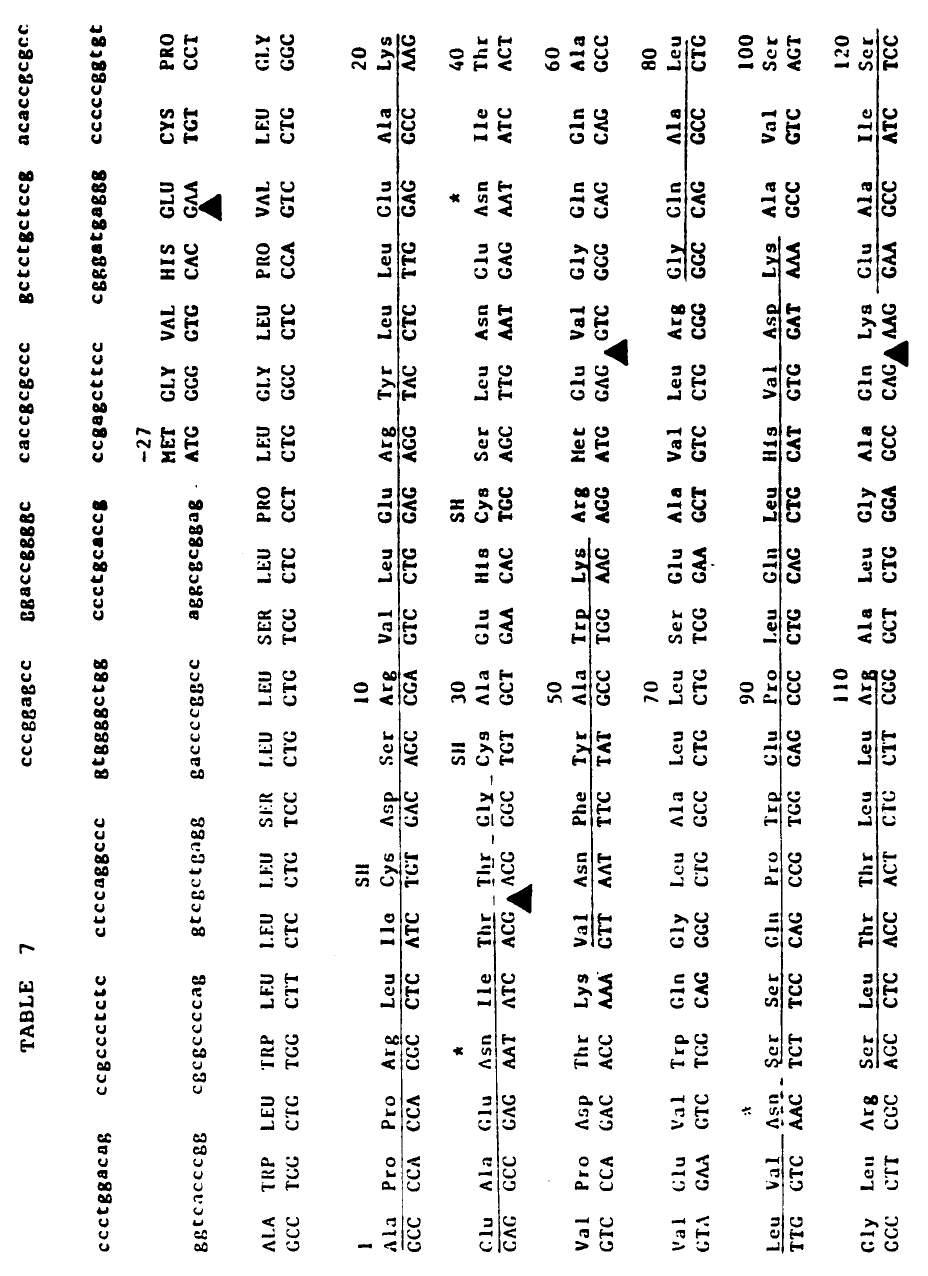

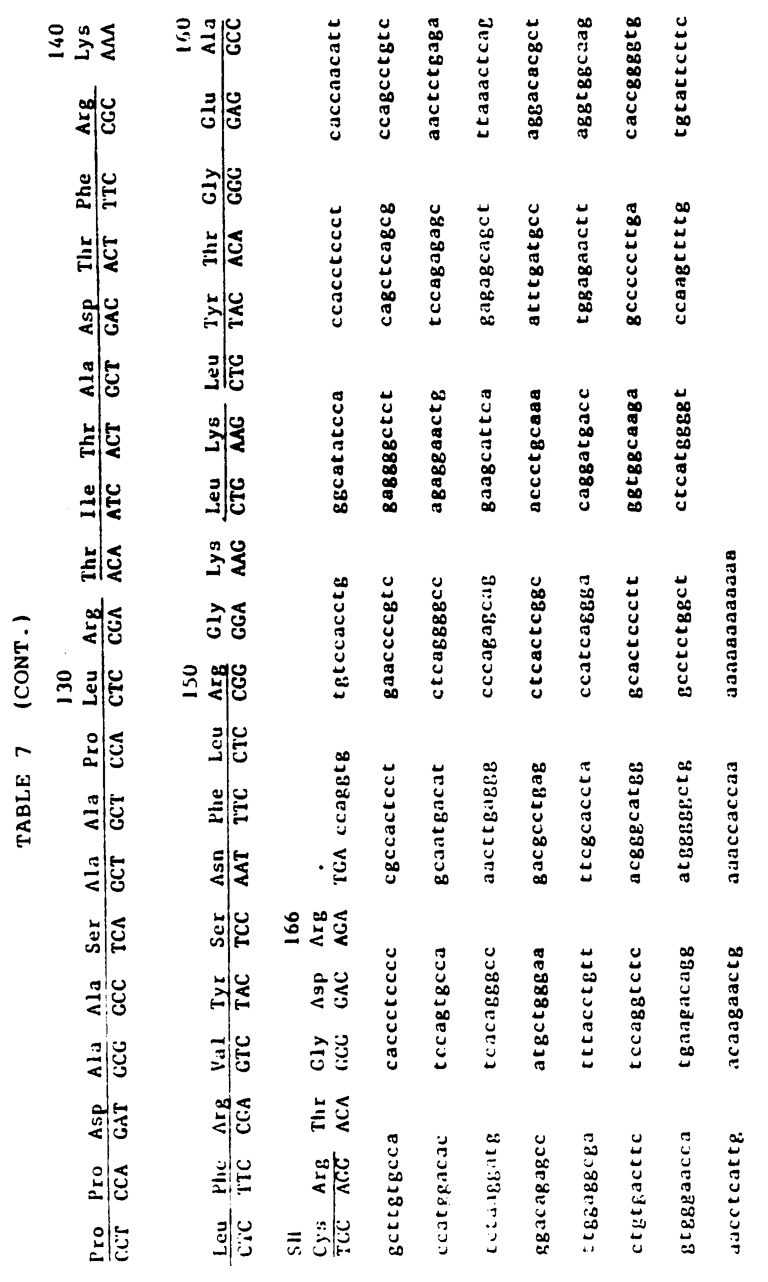

With reference to Tables 2 and 3, the deduced amino acid sequence shown below the nucleotide sequence is numbered beginning with 1 for the first amino acid of the mature protein. The putative leader peptide is indicated by all caps for the amino acid designations. Cysteine residues in the mature protein are additionally indicated by SH and potential N-linked glycosylation sites by an asterisk. The amino acids which are underlined indicate those residues identified by N-terminal protein sequencing or by sequencing tryptic fragments of EPO as described in Example 1. Partial underlining indicates residues in the amino acid sequence of certain tryptic fragments which could not be determined unambiguously. The cDNA clones lambdaHEPOFL6, lambda-HEPOFL8 and lambda-HEPOFL13 have been deposited and are available from the American Type Culture Collection, Rockville, Maryland as Accession Numbers ATCC 40156, ATCC 40152 and ATCC 40153, respectively.

### Example 4: Genomic Structure of the EPO Gene

The relative sizes and positions of four independent genomic clones (lambda-HEPO1, 2, 3, and 6) from the HaeIII/ AluI library are illustrated by the overlapping lines in Figure 3. The thickened line indicates the position of the EPO gene. A scale (in Kb) and the positions of known restriction endonuclease cleavage sites are shown. The region containing the EPO gene was completely sequenced from both strands using directed exonuclease III generated series of deletions through this region. A schematic representation of five exons coding for EPO mRNAs is shown in Figure 4. The precise 5-prime boundary of exon I is presently unknown. The protein coding portion of the exons are darkened. The complete nucleotide sequence of the region is shown in Table 4. The known limits of each exon are delineated by the solid vertical bars. Genomic clones lambda-HEPO1, lambda-HEPO2, lambda-HEPO3 and lambda HEPO6 have been deposited and are available from the American Type Culture Collection, Rockville, Maryland as Accession Numbers ATCC 40154, ATCC 40155, ATCC 40150, and ATCC 40151, respectively.

### Example 5: Construction of Vector p91023(b)

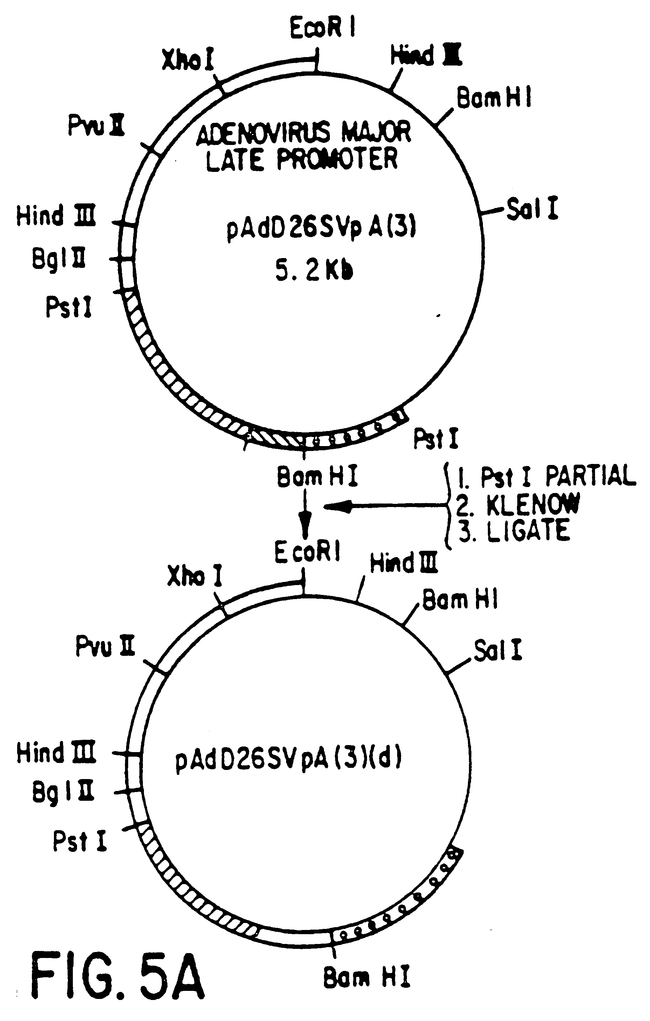

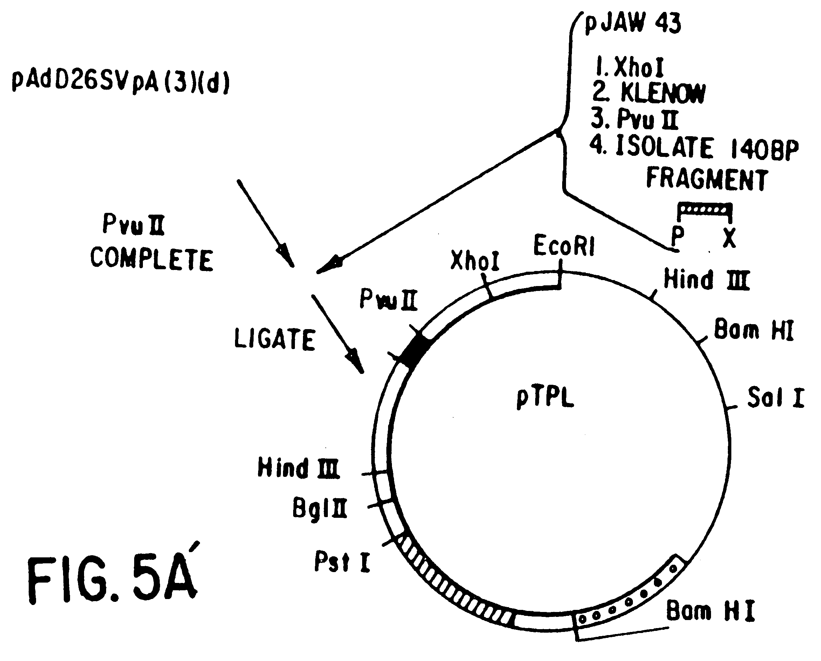

The transformation vector was pAdD26SVpA(3) described by Kaufman et al., Mol. Cell Biol., 2:1304 (1982). The structure of this vector is shown in Fig. 5A. Briefly, this plasmid contains a mouse dihydrofolate reductase (DFHR) cDNA gene that is under transcriptional control of the adenovirus 2 (Ad2) major late promoter. A 5-prime splice site is indicated in the adenovirus DNA and a 3-prime splice site, derived from an immunoglobulin gene, is present between the Ad2 major late promoter and the DFHR coding sequence. The SV40 early polyadenylation site is present downstream from the DHFR coding sequence. The procaryotic-derived section of pAdD26SVpA(3) is from pSVOd (Mellon et al., Cell, 27: 279 (1981)) and does not contain the pBR322 sequences known to inhibit replication in mammalian cells (Lusky et al., Nature, 293: 79 (1981)).

pAdD26SVpA(3) was converted to plasmid pCVSVL2 as illustrated in Fig. 5A. pAdD26SVpA(3) was converted to plasmid pAdD26SVpA(3)(d) by the deletion of one of the two Pst1 sites in pAdD26SVpA(3). This was accomplished by a partial digestion with Pst1 using a of enzyme such that a subpopulation of linearized plasmids are obtained in which only one Pst1 site was cleaved, followed by treatment with Klenow, ligation to recircularize, and screening for deletion of the Pst1 site located 3-prime to the SV40 polyadenylation sequence.

The adenovirus tripartite leader and virus associated genes (VA genes) were inserted into pAdD26SVpA(3)(d) as illustrated in Fig. 5A. First, pAdD26SVpA(3)(d) was cleaved with Pvull to make a linear molecule opened within the 3-prime portion of the three elements comprising the tripartite leader. Then, pJAW 43 (Zain et al., Cell, 16: 851 (1979)) was digested with Xho 1, treated with Klenow, digested with PvuII, and the 140bp fragment containing the second part of the third leader was isolated by electrophoresis on an acrylamide gel (6% in Tris borate buffer; Maniatis et al., supra). The 140bp fragment was then ligated to the PvuII digested pAdD26SVpA(3)(d). The ligation product was used to transform E. coli to tetracycline resistance and colonies were screened using the Grunstein-Hogness procedure employing a ³²P labelled probe hybridizing to the 140bp fragment. DNA was prepared from positively hybridizing colonies to test whether the PvuII site reconstructed was 5-prime or 3-prime of the inserted 140bp DNA specific to the second and third adenovirus late leaders. The correct orientation of the Pvull site is on the 5-prime side of the 140bp insert. This plasmid is designated tTPL in Fig. 5A.

The Ava II D fragment of SV40 containing the SV40 enhancer sequence was obtained by digesting SV40 DNA with Ava II, blunting the ends with the Klenow fragment of Pol I, ligating Xho 1 linkers to the fragments, digesting with Xho 1 to open the Xho 1 site, and isolating the fourth largest (D) fragment by gel electrophoresis. This fragment was then ligated to Xho 1 cut pTPL, yielding the plasmid pCVSVL2-TPL. The orientation of the SV40 D fragment in pCVSVL2-TPL was such that the SV40 late promoter was in the same orientation as the adenovirus major late promoter.

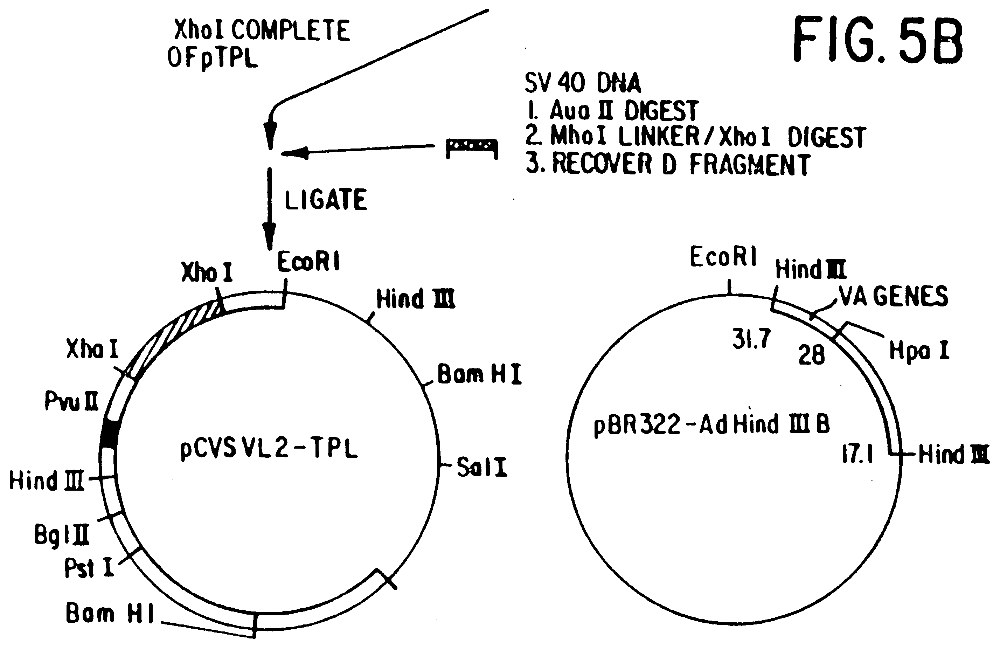

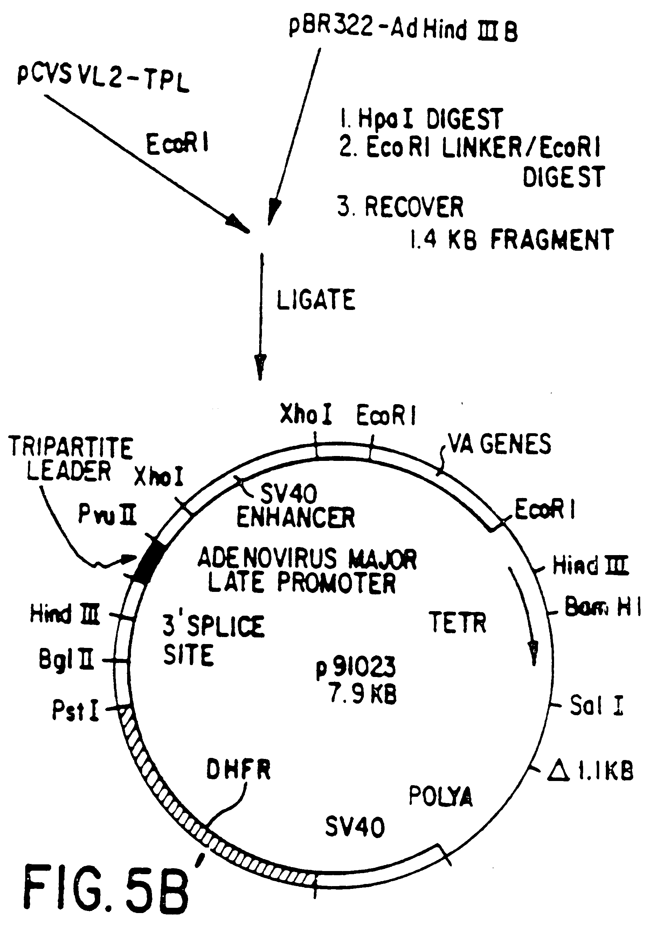

To introduce the adenovirus associated (VA) genes into the pCVSVL2-TPL, first a plasmid pBR322 was constructed that contained the adenovirus type 2 Hind III B fragment. Adenovirus type 2 DNA was digested with Hind III and the B fragment was isolated by gel electrophoresis. This fragment was inserted into pBR322 which had previously been digested with Hind III. After transformation of E. coli to ampicillin resistance, recombinants were screened for insertion of the Hind III B fragment and the inserted orientation was determined by restriction enzyme digestion. pBR322 - Ad Hind III B contains the adenovirus type 2 Hind III B fragment in the orientation depicted in Fig. 5B.

As illustrated in Fig. 5B, the VA genes are conveniently obtained from plasmid pBR322 - Ad Hind III B by digestion with Hpa I, adding EcoR1 linkers and digestion with EcoR1, followed by recovery of the 1.4kb fragment. The fragment having EcoR1 sticky ends is then ligated into the EcoR1 site of PTL, previously digested with EcoR1. After transforming E. coli HB101 and selecting for tetracycline resistance, colonies were screened by filter hybridization to DNA specific for the VA genes. DNA was prepared from positively hybridizing clones and characterized by restriction endonuclease digestion. The resulting plasmid is designated p91023.

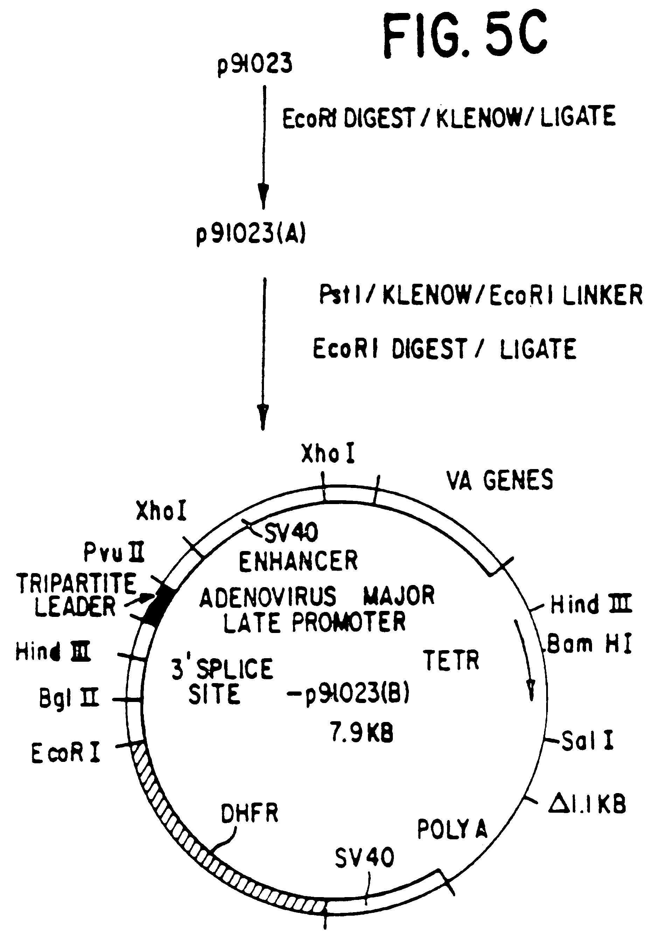

As illustrated in Fig. 5C, the two EcoR1 sites in p91023 were removed by cutting p91023 to completion with EcoR1, generating two DNA fragments, one about 7kb and the other about 1.3kb. The latter fragment contained the VA genes. The ends of both fragments were filled in using the Klenow fragment of PolI and the two fragments were then ligated together. A plasmid p91023(A), containing the VA genes and similar to p91023, but deleted for the two EcoR1 sites, were identified by Grunstein-Hogness screening with the Va gene fragment, and by conventional restriction site analysis.

The single Pst1 site in p91023(A) was removed and replaced with an EcoR1 site. p91023(a) was cut to completion with Pst1 and treated with the Klenow fragment of PolI to generate flush ends. EcoR1 linkers were ligated to the blunted Pst1 site of p91023(A). The linear p91023(A), with EcoR1 linkers attached at the blunted Pst1 site was separated from unligated linkers and digested to completion with EcoR1, and religated. A plasmid, p91023(B) as depicted in Figure SC was recovered, and identified as having a structure similar to p91023(A), but with an EcoR1 site in place of the former Pst1 site. Plasmid p91023(B) has been deposited and is available from the American Type Culture Collection, Rockville, Maryland as Accession Number ATCC 39754.

### Example 6:

The cDNA clones (lambda-EPOFL6 and lambda-EPOFL13: Example 3) were inserted into the plasmid p91023(B) forming pPTFL6 and pPTFL13, rspectively. 8 ug of each of the purified DNA's was then used to transfect 5 x 10⁶ COS cells using the DEAE-dextran method (infra). After 12 hrs., the cells were washed and treated with Chloroquin (0.1mM) for 2 hrs., washed again, and exposed to 10 ml media containing 10% fetal calf serum for 24 hrs. The media was changed to 4 ml serum free media and harvested 48 hrs. later.

Production of immunologically active EPO was quantified by a radioimmunoassay as described by Sherwood and Goldwasser (55). The antibody was provided by Dr. Judith Sherwood. The iodinated tracer was prepared from the homogeneous EPO described in Example 1. The sensitivity of the assay is approximately 1ng/ml. The results are shown below in Table 8.

**TABLE 8**

| VECTOR | LEVEL OF EPO RELEASED INTO THE MEDIA (ng/ml) |
|---|---|
| pPTFL13 | 330 |
| pPTFL6 | 31 |

PTFL13 has been deposited and is available from the American Type Culture Collection, Rockville, Maryland under Accession No. ATCC 39990.

### Example 7

EPO cDNA (lambda-HEPOFL13) was inserted into the p91023(B) vector and was transfected into COS-1 cells and harvested as described above (Example 6) except that the chloroquin treatment was omitted.

In vitro biologically active EPO was measured using either a colony forming assay with mouse fetal liver cells as a source of CFU-E or a ³H-thymidine uptake assay using spleen cells from phenylhydrazine injected mice. The sensitivities of these assays are approximately 25 mUnits/ml. In vivo biologically active EPO was measured using either the hypoxic mouse or starved rat method. The sensitivity of these assays is approximately 100 mU/ml. No activity was detected in either assay from mock condition media. The results of EPO expressed by clone EPOFL13 are shown below in Table 9 wherein the activities reported are expressed in units/ml, using a commercial, quantified EPO (Toyobo, Inc.) as a standard.

**TABLE 9**

| EPO Excreted from COS Cells Transfected with Type I EPO cDNA | | |
|---|---|---|
| Assay | Activity | |
| RIA | 100 | ng/ml |
| CFU-E | 2 | 0.5 U/ml |
| ³H-Thy | 3.1 | 1.8 U/ml |
| hypoxic mouse | 1 | U/ml |
| starved rat | 2 | U/ml |

### Example 8: SDS Polyacrylamide Gel Analysis of EPO from COS Cells

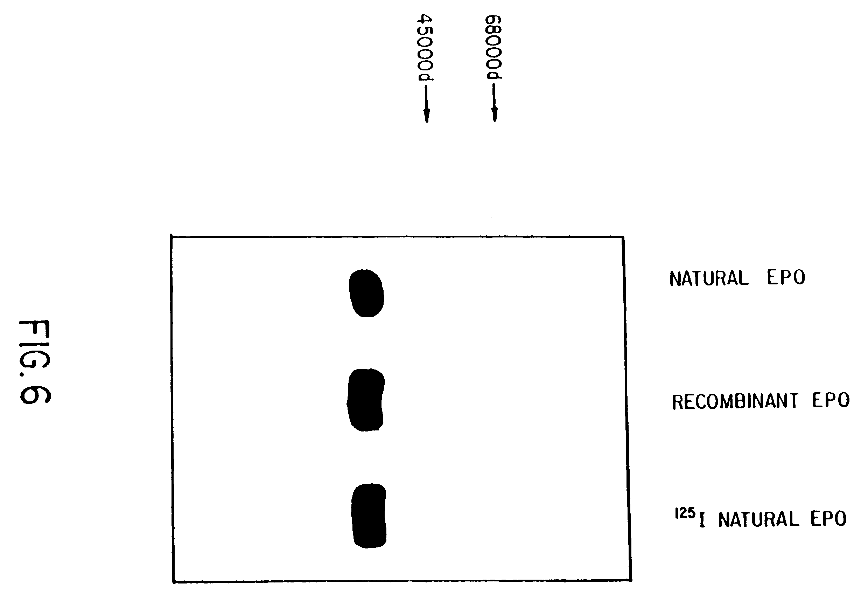

180 ng of EPO released into the media of COS cells transfected with EPO (lambda-HEPOFL13) cDNA in the vector 91023(B) (supra) was electrophoresed on a 10% SDS Laemlli polyacrylamide gel and electrotransferred to nitrocellulose paper (Towbin et al., Proc. Natl. Acad. Sci. USA 76:4350 (1979)). The filter was probed with anti-EPO antibody as described in Table 8, washed, and reprobed with ¹²⁵I-staph A protein. The filter was autoradiographed for two days. Native homogeneous EPO was described in Example 1, either before (lane B) or after iodination (lane C) were electrophoresed (see Figure 6). Markers used included ³⁵S methionine labelled, serum albumin (68,000 d) and ovalbumin (45,000 d).

### Example 9: Construction of RK1-4

The Bam HI-PvuII fragment from the plasmid PSV2DHFR (Subramani et al., Mol. Cell. Biol. 1:854-864 (1981)) containing the SV40 early region promoter adjacent to the mouse dihydrofolate reductase (DHFR) gene, an SV40 enhancer, the small t antigen intron, and the SV40 polyadenylation sequence was isolated (fragment A). The remaining fragments were obtained from the vector p91023(A) (supra) as follows: p91023(A) was digested with Pst I at the single Pst I site near to the adenovirus promoter to linearize the plasmid and either ligated to synthetic Pst I to EcoRI converters and recircularized (creating the sites Pst I - EcoRI - Pst I at the original Pst I site; 91023(B') or treated with the large fragment of DNA polymerase I to destroy the Pst I sites and ligated to a synthetic EcoRI linker and recircularized (creating an EcoRI site at the original Pst I site; 91023(B). Each of the two resulting plasmids 91023(B) and 91023(B') were digested with Xba and EcoRI to produce two fragments (F and G). By joining fragment F from p91023(B) and fragment G from p91023(B') and fragment G from p91023(B) and fragment F from p91023(B') two new plasmids were created which contained either an EcoRI - Pst I site or a Pst I - EcoRI site at the original Pst I site. The plasmid containing the Pst I - EcoRI site where the Pst I site is closest to the adenovirus major late promoter was termed p91023(C).

The vector p91023(C) was digested with XhoI to completion and the resulting linearized DNA with sticky ends was blunted by an end filling reaction with the large fragment of E. coli of DNA polymerase I. To this DNA was ligated a 340 bp Hind III - EcoRI fragment containing the SV40 enhancer prepared as follows:

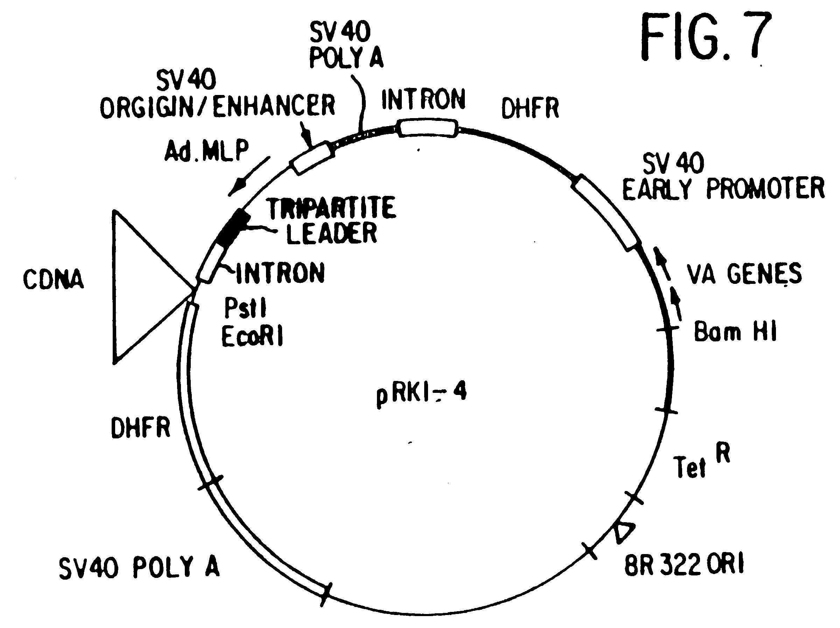

The Hind III - Pvu II fragment from SV40 which contains the SV40 origin or replication and the enhancer was inserted into the plasmid c lac (Little et al., Mol. Biol. Med. 1:473-488 (1983)). The c lac vector was prepared by digesting c lac DNA with BamHI, filling in the sticky ends with the large fragment of DNA polymerase I and digesting the DNA with Hind III. The resulting plasmid (c SVHPlac) regenerated the BamHI site by ligation to the Pvu II blunt end. The EcoRI - Hind III fragment was prepared from c SVHPlac and ligated to the EcoRI - Hind III fragment of PSVOd (Mellon et al., supra) which contained the plasmid origin of replication and the resulting plasmid pSVHPOd was selected. The 340 bp EcoRI - Hind III fragment of PSVHPOd containing the SV40 origin/enhancer was then prepared, blunted at both ends with the large fragment of DNA polymerase I, and ligated to the Xho1 digested, blunted p91023(c) vector described above. The resulting plasmid (p91023 (C)/Xho/blunt plus EcoRI/Hind III/blunt SV40 origin plus enhancer) in which the orientation of the Hind III - EcoRI fragment was such that the BamHI site within that fragment was nearest to the VA gene was termed pES105. The plasmid pES105 was digested with Bam HI and PvuII and also with PvuII alone and the BamHI -PvuII fragment containing the adenovirus major late promoter (fragment B) and the PvuII fragment containing the plasmid during resistance gene (tetracycline resistance) and other sequences (fragment C) were isolated. Fragments A, B and C were ligated and the resulting plasmid shown in Figure 7 was isolated and termed RK1-4. Plasmid RK1-4 has been deposited with the American Type Culture Collection, Rockville, Maryland, where it is available under Accession Number ATCC 39940.

### Example 10: Expression of EPO in CHO cells-Method I

DNA (20 ug) from the plasmid pPTFL13 described above (Example 6) was digested with the restriction endonuclease Cla I to linearize the plasmid and was ligated to Cla I-digested DNA from the plasmid pAdD26SVp(A) 1 (2 ug) which contains an intact dihydrofolate reductase (DHFR) gene driven by an adenovirus major late promoter (Kaufman and Sharp, Mol. and Cell Biol. 2:1304-1319 (1982)). This ligated DNA was used to transfect DHFR-negative CHO cells (DUKX-BII, Chasin L.A. and Urlaub G. (1980) PNAS 77 4216-4220) and following growth for two days, cells which incorporated at least one DHFR gene were selected in alpha media lacking nucleotides and supplemented with 10% dialyzed fetal bovine serum. Following growth for two weeks in selective media, colonies were removed from the original plates, pooled into groups of 10-100 colonies per pool, replated and grown to confluence in alpha media lacking nucleotides. The supernatant media from the pools grown prior to methotrexate selection were assayed for EPO by RIA. Pools which showed positive EPO production were grown in the presence of methotrexate (0.02 uM) and then subcloned and reassayed. EPO Cla 4 4.02-7, a single subcloned from the EPO Cla 4 4.02 pool, releases 460 ng/ml EPO into media containing 0.02 uM MTX (Table 10). EPO Cla 4 4.02-7 is the cell line of choice for EPO production and has been deposited with the American Type Culture Collection as Accession Number ATCC CRL8695. Currently, this clone is being subjected to stepwise selection in increasing concentrations of MTX, and will presumably yield cells which produce even higher levels of EPO. For pools which were negative by RIA, methotrexate resistant colonies obtained from the counterpart cultures which were grown in the presence of methotrexate (0.02 uM) were again reassayed in pools for EPO by RIA. Those cultures which were not positive were subcloned and subjected to growth in further increasing concentrations of methotrexate.

Stepwise methotrexate (MTX) selection was achieved by repeated cycles of culturing the cells in the presence of increasing concentrations of methotrexate and selecting for survivors. At each round, EPO was measured in the culture supernatant by RIA and by in vitro biological activity. The levels of methotrexate used in each stepwise amplification were 0.02 uM, 0.1 uM, and .5 uM. As shown in Table 10 after 1 round of selection in .02 uM MTX significant levels of EPO were being released into the culture media.

**TABLE 10**

| Level of EPO Released into the Media | | | | |
|---|---|---|---|---|
| Sample | | Assay | Alpha medium harvest | 0.02 uM methotrexate in alpha medium harvest |
| 4 4 | Pool | RIA | 17 ng/ml | 50 ng/ml |
| 4 4 | Single Colony | | | |
| | Clone (.02-7) | RIA | | 460 ng/ml |

### Example 11: Expression of EPO in CHO cells - Method II

DNA from the clone lambda HEPOFL13 was digested with EcoRI and the small RI fragment containing the EPO gene was subcloned into the EcoRI site of the plasmid RK1-4 (See Example 10). This DNA (RKFL13) was then used to transfect the DHFR-negative CHO cells directly (without digestion) and the selection and amplification was carried out as described in Example 10 above.

The RKFL13 DNA was also inserted into CHO cells by protoplast fusion and microinjection. Plasmid RKFL13 has been deposited and is available from the American Type Culture Collection, Rockville, Maryland under Accession No. ATCC 39989.

**TABLE 11**

| Level of EPO Released into the Media | | | |
|---|---|---|---|
| Sample | Assay | alpha medium harvest | 0. 02uM methotrexate in alpha medium harvest |
| Colony Pool A | RIA | 3 ng/ml | 42 ng/ml (pool) |
| | | | 150 ng/ml (clone) |
| | ³H-Thy | -- | 1.5 U/ml |
| Single Colony clone(.02C-Z) | RIA | -- | 90 ng/ml |
| | ³H-Thy | -- | 5.9 U/ml |
| Microinjected pool (DEPO-l) | RIA | 60 ng/ml | 160 ng/ml |
| | ³H-Thy | 1.8 U/ml | -- |

The preferred single colony clone has been deposited and is available from the American Type Culture Collection, Rockville, Maryland under Accession Number ATCC CRL8695.

### Example 12: Expression of EPO Genomic Clone in COS-1 Cells

The vector used for expression of the EPO genomic clone is pSVOd (Mellon et al., supra). DNA from pSVOD was digested to completion with Hind III and blunted with the large fragment of DNA polymerase I. The EPO genomic clone lambda-HEPO3 was digested to completion with EcoRI and Hind III and the 4.0 kb fragment containing the EPO gene was isolated and blunted as above. The nucleotide sequence of this fragment from the Hind III site to a region just beyond the polyadenylation signal is shown in Figure 4 and Table 4. The EPO gene fragment was inserted into the pSVOd plasmid fragment and correctly constructed recombinants in both orientations were isolated and verified. The plasmid CZ2-1 has the EPO gene in orientation "a" (i.e. with the 5' end of EPO nearest to the SV40 origin) and the plasmid CZ1-3 is in the opposite orientation (orientation "b").

The plasmids CZ1-3 and CZ2-1 were transfected into COS-1 cells as described in Example 7 and media was harvested and assayed for immunologically reactive EPO. Approximately 31 ng/ml of EPO was detected in the culture supernatant from CZ2-1 and 16-31 ng/ml from CZ1-3.

Genomic clones HEPO1, HEPO2, and HEPO6 can be inserted into COS cells for expression in a similar manner.

### Example 13: Expression in C127 and in 3T3 Cells Construction of pBPVEPO

A plasmid containing the EPO cDNA sequence under the transcriptional control of a mouse metallothionein promoter and linked to the complete bovine papilloma virus DNA was prepared as follows:

### pEPO49f

The plasmid SP6/5 was purchased from Promega Biotec. This plasmid was digested to completion with EcoR1 and the 1340 bp EcoR1 fragment from lambda-HEPOFL13 was inserted by DNA ligase. A resulting plasmid in which the 5' end of the EPO gene was nearest to the SP6 promoter (as determined by BglI and Hind III digestion) was termed pEPO49F. In this orientation, the BamHI site in the PSP6/5 polylinker is directly adjacent to the 5' end of the EPO gene.

### pMMTneo BPV

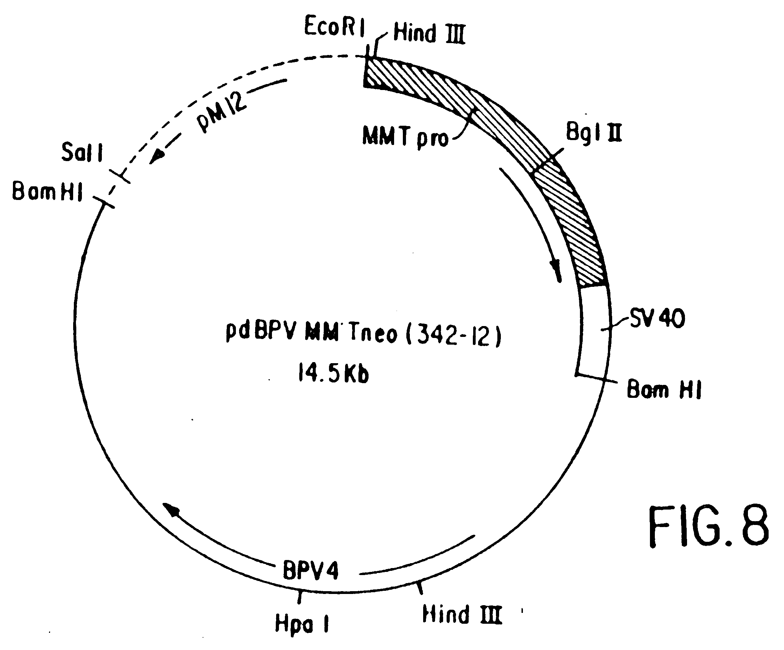

The plasmid pdBPV-MMTneo (342-12) (Law et al., Mol. and Cell Biol. 3:2110-2115 (1983)), illustrated in Figure 8, was digested to completion with BamHI to produce two fragments - a large fragment -8kb in length containing the BPV genome and a smaller fragment, -6.5 kb in length, containing the pML2 origin of replication and ampicillin resistance gene, the metallothionein promoter, the neomycin resistance gene, and the SV40 polyadenylation signal. The digested DNA was recircularized by DNA ligase and plasmids which contained only the 6.8 kb fragment were identified by EcoRI and BamHI restrictions endonuclease digestion. One such plasmid was termed pMMTneo BPV.

### pEPO15a

pMMTneo BPV was digested to completion with BglII. pEPO49f was digested to completion with BamHI and BglII and the approximately 700 bp fragment containing the entire EPO coding region was prepared by gel isolation. The BglII digested pMMTneo BPV and the 700 bp BamHI/BglII EPO fragment were ligated and resulting plasmids containing the EPO cDNA were identified by colony hybridization with an oligonucleotide d(GGTCATCTGTCCCCTGTCC) probe which is specific for the EPO gene. Of the plasmids which were positive by hybridization analysis, one (pEPO15a) which had the EPO cDNA in the orientation such that the 5' end of the EPO cDNA was nearest to the metallothionein promoter was identified by digestion with EcoRI and KpnI.

### pBPV-EPO

The plasmid pEPO15A was digested to completion with BamHI to linearize the plasmid. The plasmid pdBPV-MMTneo(342-12) was also digested to completion with BamHI to produce two fragments of 6.5 and 8kb . The 8kb fragment which contained the entire Bovine Papilloma Virus genome, was gel isolated. pEPO15a/BamHI and the 8kb BamHI fragment were ligated together and a plasmid (pBPV-EPO) which contained the BPV fragment was identified by colony hybridization using an oligonucleotide probe d(P-CCACACCCGGTACACA-OH) which is specific for the BPV genome. Digestion of pBPV-EPO DNA with Hind III indicated that the direction of transcription of the BPV genome was the same as the direction of transcription from the metallothionein promoter (as in pdBPV-MMTneo(342-12) see Figure 8). The plasmid pdBPV-MMTneo(342-12) is available from the American Type Culture Collection, Rockville, Maryland under Accession No. ATCC 37224.

### Expression

The following methods were used to express EPO.

### Method I.

DNA pBPV-EPO was prepared and approximately 25 ug was used to transfect ~1x 10⁶ C127 (Lowy et al., J. of Virol. 26:291-98 (1978)) CHO cells using standard calcium phosphate precipitation techniques (Grahm et al., Virology, 52:456-67 (1973)). Five hrs. after transfection, the transfection media was removed, the cells were glycerol shocked, washed, and fresh α-medium containing 10% fetal bovine serum was added. Forty-eight hrs. later, the cells were trypsinized and split at a ratio of 1:10 in DME medium containing 500 ug/ml G418 (Southern et al., Mol. Appl. Genet. 1:327-41 (1982)) and the cells were incubated for two-three weeks. G418 resistant colonies were then isolated individually into microtiter wells and grown until sub-confluent in the prsence of G418. The cells were then washed, fresh media containing 10% fetal bovine serum was added and the media was harvested 24 hours later. The conditioned media was tested and shown to be positive for EPO by radioimmunoassay and by in vitro biological assay:

### Method II

C127 or 3T3 cells were cotransfected with 25ug of pBPV-EPO and 2ug of pSV2neo (Southern et al., supra) as described in Method I. This is approximately at 10-fold molar excess of the pBPV-EPO. Following transfection, the procedure is the same as in Method I.

### Method III

C127 cells were transfected with 30 ug of pBPV-EPO as described in Method I. Following transfection and splitting (1:10), fresh media was exchanged every three days. After approximately 2 weeks, foci of BPV transformed cells were apparent. Individual foci were picked separately into 1 cm wells of a microtiter plate, grown to a sub-confluent monolayer and assayed for EPO activity or antigenicity in the conditioned media.

### Example 14: Expression in Insect cells Construction of pIVEV EPOFL13

The plasmid vector pIVEV has been deposited and is available from the American Type Culture Collection, Rockville, Maryland under Accession No. ATCC 39991. The vector was modified as follows:

### pIVEVNI

pIVEV was digested with EcoRI to linearize the plasmid, blunted using the large fragment of DNA polymerase I and a single NotI linker
GGCGGCCGCC
CCGCCGGCGG
was inserted by blunt end ligation. The resultant plasmid is termed pIVEVNI.

### pIVEVSI

pIVEV was digested with SmaI to linearise the plasmid and a single SfiI linker
GGGCCCCAGGGGCCC
CCCGGGGTCCCCGGG
was inserted by blunt end ligation. The resultant plasmid was termed pIVEVSI.

### pIVEVSIBgKp

The plasmid pIVEVSI was digested with KpnI to linearize the plasmid and approximately 0 to 100 bp were removed from each end by digestion with the double-stranded exonuclease Bal 31. Any resulting ends which were not perfectly blunt were blunted using the large fragment of DNA polymerase I and the polylinker 
Xho I XbaI
BglII EcoRI ClaI KpnI
AGATCTCGAGAATTCTAGATCGATGGTACC
TCTAGAGCTCTTAAGATCTAGCTACCATGG
 was inserted by blunt end ligation. The polylinker was inserted in both orientations. A plasmid in which the polylinker is oriented such that the BglII site within the polylinker is nearest to the polyhedron gene promoter is termed pIVEVSIBgKp. A plasmid in which the KpnI site within the polylinker is nearest to the polyhedron gene promoter is termed pIVEVSIKpBg. The number of base pairs which were deleted between the original KpnI site in pIVEVSI and the polyhedron promoter was not determined. The pIEIVSIBgKp has been deposited with and is available from the American Type Culture Collection, Rockville, Maryland under Accession No. ATCC 39988.

### pIEVSIBgKpNl

pIVEVNI was digested to completion with KpnI and PstI to produce two fragments. The larger fragment, which contained the plasmid origin of replication and the 3' end of the polyhedron gene was prepared by gel isolation (fragment A). pIVEVSIBgKp was digested to completion with PstI and Kpn to produce two fragments and the smaller fragment, which contained the polyhedron gene promoter and the polylinker was prepared by gel isolation (fragment B). Fragment A and B were then joined by DNA ligase to form the new plasmid pIVEVSIBgKpNl which contains a partially deleted polyhedron gene into which a polylinker has been inserted and also contains a NotI site (replacing the destroyed EcoRI site) and a SfiI site which flank the polyhedron gene region.

### pIVEPO

pIVEVSI BGKpNI was digested to completion with EcoRI to linearize the plasmid and the 1340 bp EcoRI fragment from lambda-HEPOFL13 was inserted. Plasmids containing the EPO gene in the orientation such that the 5' end of the EPO gene is nearest to the polyhedron promoter and the 3' end of the polyhedron gene were identified by digestion with BglII. One of these plasmids in the orientation described above was designated pIVEPO.

### Expression of EPO in Insect CElls

Large amounts of the pIVEPO plasmid were made by transforming the E. coli strain JM101-tgl. The plasmid DNA was isolated by cleared lysate technique (Maniatis and Fritsch, Cold Spring Harbor Manual) and further purified by CsCl centrifugation. Wild-type Autographa californica polyhedrosis virus (AcNPV) strain L-1 DNA was prepared by phenol extraction of virus particles and subsequent CsCl purification of the viral DNA.

These two DNAs were then cotransfected into Spodoptera frugiperda cells IPLB-SF-21 (Vaughn et al., In Vitro Vol. B, pp. 213-17 (1977) using the calcium phosphate transfection procedure (Potter and Miller, 1977). For each plate of cells being cotransfected, lug of wild-type AcNPV DNA and 10 ug of pIVEPO were used. The plates were incubated at 27°C for 5 days. The supernatant was then harvested and EPO expression in the supernatant was confirmed by radioimmunoassay and by in vitro biological assay.

### Example 15: Purification of EPO

COS-cell conditioned media (121) with EPO concentrations up to 200ug/litre was concentrated to 600ml using 10,000 molecular weight cutoff ultrafiltration membranes, such as a Millipore Pellican® fitted with 5 sq. ft. of membrane. Assays were performed by RIA as described in Example 6. The retentate from the ultrafiltration was diafiltered against 4ml. of 10mM sodium phosphate buffered at pH7.0. The concentrated and diafiltered condition media contained 2.5mg of EPO in 380mg of total protein. The EPO solution was further concentrated to 186ml and the precipitated proteins were removed by centrifugation at 110,000 xg for 30 minutes.

The supernatant which contained EPO (2.0mg) was adjusted to pH5.5 with 50% acetic acid, allowed to stir at 4°C for 30 minutes and the precipitate removed by centrifugation at 13,000 xg for 30 minutes.

### Carbonylmethyl Sepharose Chromatography

The supernatant from the centrifugation (20ml) containing 200ug of EPO (24mg total protein) was applied to a column packed with CM-Sepharose (20ml) equilibrated in 10mM sodium acetate pH5.5, washed with 40ml of the same buffer. EPO which bound to the CM-Sepharose was eluted with a 100ml gradient of NaU(0-1) in 10mM sodium phosphate pH5.5. The fractions containing EPO (total of 50ug in 2mg of total proteins) were pooled and concentrated to 2ml using Amicon YM10 ultrafiltration membrane.

### Reverse phase-HPLC

The concentrated fractions from CM-Sepharose containing the EPO was further purified by reverse phase-HPLC using Vydac C-4 column. The EPO was applied onto the column equilibrated in 10% solvent B (Solvent A was 0.1% CF₃CO₂H in water; solvent B was 0.1% CF₃CO₂H in CF₃CN) at flow rate of 1ml/min. The column was washed with 10%B for 10 minutes and the EPO was eluted with linear gradient of B (10-70% in 60 minutes). The fractions containing EPO were pooled (~40ug of EPO in 120ug of total proteins) and lyophilized. The lyophilized EPO was reconstituted in 0.1M Tris-HCl at pH7.5 containing 0.15M NaCl and rechromatographed on the reverse phase HPLC. The fractions containing the EPO were pooled and analyzed by SDS-polyacrylamide (10%) gel electrophoresis (Lameli, U.K., Nature). The pooled fractions of EPO contained 15.5ug of EPO in 25ug of total protein.

### REFERENCES

1) Jacobson, L. O., Goldwasser, E. Fried, W., and Plzak, L. F., Trans. Assoc. Am. Physicians TO:305-317 (1957).
2) Krantz, S. B. and Jacobson, L. O. Chicago: University of Chicago Press 1970, pp. 29-31.
3) Hammond, D and Winnick, S. Ann. N.Y. Acad. Sci. 230:219-227 (1974).
4) Sherwood, J. B. and Goldwasser, E., Endocrinology 103:866-870 (1978).
5) Fried, W. Blood 40:671-677 (1972).
6) Fisher, J. J. Lab. and Clin. Med. 93:695-699 (1979).
7) Naughton, B. A., Kaplan, S. M., Roy, M., Burdowski, A. J., Gordon, A. S., and Piliero, S. J. Science 196:301-302.
8) Lucarelli, G. P., Howard, D., and Stohlman, F., Jr. J. Clin. Invest 43:2195-2203 (1964).
9) Zanjani, E. D., Poster, J., Burlington, H., Mann, L. I., and Wasserman, L. R. J. Lab. Clin. Med. 89:640-644 (1977).
10) Krantz, S. B., Gallien-Lartigue, O., and Goldwasser, E. J. Biol Chem. 238:4085-4090 (1963).
11) Dunn, C. D., Jarvis, J. H. and Greenman, J. M. Exp. Hematol. 3:65-78 (1975).
12) Krystal, G. Exp. Hematol. 11:649-660 (1983)
13) Iscove, N.N. and Guilbert, L.J., M.J. Murphy, Jr. (Ed.) New York:Springer-Verlag, pp. 3-7 (1978).
14) Goldwasser, E., ICN UCLA Symposium, Control of Cellular Division and Development, A. R. Liss, Inc., pp. 487-494 (1981)
15) Cline, M. J. and Golde, D. W. Nature 277:177-181 (1979)
16) Metcalf, D., Johnson, G. R., and Burgess, A. W. Blood 55:138- (1980)
17) Krane, N. Henry Ford Hosp. Med. J. 31:177-181 (1983)
18) Eschbach, J., Madenovic, J., Garcia, J., Wahl, P., and Adamson, J. J. Clin. Invest. 74;434-441 (1984)
19) Anagnostou, A., Barone, J., Vedo, A., and Fried, W. Br. J. Hematol 37:85-91 (1977)
20) Miyake, T., Kung, C., and Goldwasser, E. J. Biol. Chem. 252:5558-5564 (1977)
21) Yanagawa, S., Hirade, K., Ohnota, H., Sasaki, R., Chiba, H., Veda, M., and Goto, M. J. Biol. Chem. 259:2707-2710 (1984)
22) Lawn, R. M., Fritsch, E. F., Parker, R. C., Blake, G., and Maniatis, T. Cell 15:1157 - (1978)
23) Sanger, F., Nicklen, S., and Coulson, A. R. Proc. Nat'l. Acad. Sci., U.S.A. 74:5463- (1977)
24) Zanjanc, E.D., Ascensao, J.L., McGlave, P.B., Banisadre, M., and Ash, R. C. J. Clin. Invest. 67:1183- (1981)
25) Toole, J.J., Knopf, J.L., Wozney, J.M., Sultzman, L.A. Buecker, J. L., Pittman, D. D., Kaufman, R. J., Brown, E., Shoemaker, C., Orr, E. C., Amphlett, G. W., Foster, W. B., Coe, M. L., Knutson, G. J., Fass, D. N., and Hewick, R. M. Nature in Press
26) Goldwasser, E. Blood Suppl. 1, 58, xlii (abstr) (1981)
27) Sue, J. M. and Sytkowdki, A. J. Proc. Nat'l Acad. Sci U.S.A. 80:3651-3655 (1983)
29) Bersch, N. and Golda, D.W., In Vitro Aspects of Erythropoiesis. M. J. Murphy (Ed.) New York:Springer-Verlag (1978)
30) Cotes, P. M. and Bangham, D. R. Nature 191:1065- (1961)
31) Goldwasser, E. and Gross, M. Methods in Enzymol 37:109-121 (1975)
32) Nabeshima, Y. -i, Fujii-Kuriyama, Y., Muramatsu, M., and Ogata, K. Nature 308:333-338 (1984)
33) Young, R. A., Hagencuhle, O. and Schibler, U. Cell 23:451-558 (1981)
34) Medford, R. M., Nguyen, H. T., Destree, A. T., Summers, E. and Nadal-Ginard, B. Cell 38:409-421 (1984)
35) Ziff, E. B. Nature 287:491-499 (1980)
36) Early, P. Cell 20:313-319 (1980)
37) Sytkowski, A. Bio. Biop. Res. Comm. 96:143-149 (1980)
38) Murphy, M. and Miyake, T. Acta. Haematol. Jpn. 46:1380-1396 (1983)
39) Wagh, P. V. and Bahl, O. P. CRC Critical Reviews in Biochemistry 307-377 (1981)
40) Wang, F. F., Kung, C. K. -H. and Goldwasser, E. Fed. Proc. Fed. Am. Soc. Exp-. Biol. 42:1872 (abstr) (1983)
41) Lowy, P., Keighley, G. and Borsook, H. Nature 185:102-103 (1960)
42) VanLenten, L. and Ashwell, G. J. Biol. Chem. 247:4633-4640 (1972)
43) Lee-Huang, S. Proc. Nat'l Acad. Sci. U.S.A. 81:2708-2712 (1984)
44) Fyhrquist, F., Rosenlof, K., Gronhagen-Riska, C., Hortling, L. and Tikkanen, I. Nature 308:649-562 (1984)
45) Ohkubo, H., Kageyama, R., Vjihara, M., Hirose, T., Inayama, S., and Nakanishi, S. Proc. Nat'l Acad. Sci. U.S.A. 80:2196-2200 (1983)
46) Suggs, S.V., Wallace, R. B., Hirose, T., Kawashima, E. H. and Itakura, K. Proc. Nat'l. Acad. Sci. U.S.A.-78:6613-6617 (1981)
47) Woo, S. L. C., Dugaiczyk, A., Tsai, M. -J., Lai, E. C., Catterall, J. F. and O'Malley, B. W. Proc. Nat'l.-Acad. Sci. U.S.A. 75:3688- (1978)
48) Melchior, W. B. and VonHippel, P. H. Proc. Nat'l Acad. Soc. U.S.A. 70:298-302 (1973)
49) Orosz, J. M. and Wetmis, J. G. Biopolymers 16:1183-1199 (1977)
50) Anderson, S and Kingston, I. B. Proc. Nat'l Acad. Sci.-U.S.A. 80:6836-6842 (1983)
51) Ullrich, A., Coussens, L., Hayflick, J. S. Dull, T. J., Gray, A., Tam, A. W., Lee, J., Yarden, Y., Libermann, T. A., Schlessinger, J., Downward, J., Mayes, E. L. V., Whittle, H., Waterfield, M.D. and Seeburg, P. H. Nature 309: 418-425 (1984)
52) Fisher, J. Proc. Soc. Exptl. Biol. and Med. 173:289-305 (1983)
53) Kozak, M. Nuc. Acid Res. 12:857-872 (1984)
54) Benton, W.D. and Davis, R.W. Science 196:180-182 (1977)
55) Sherwood, J.B. and Goldwasser, E. Blood 54:885-893 (1979)
56) Derman, E., Krauter, K., Walling, L., Weinberger, C., Ray, M., and Darnell, J. T., Cell 23:731- (1981)
57) Gluzman, Y., Cell 23:175-182 (1981)
58) Hewick, R. M., Hunkapiller, M. E., Hood, L. E., and Dreyer, W. J. J. Biol. Chem. 256:7990-7997 (1981)
59) Towbin, H., Stachelin, T., and Gordon, J., Proc. Nat'l Acad. Sci. 76:4380- (1979)
60) Carnott, P., DeFlandre, C. C. R. Acad. Sci. Paris 143:432-(1960)

## Claims

1. A method for the production of human erythropoietin comprising culturing in a suitable medium eukaryotic host cells containing the DNA sequence as shown in Table 3 from the sequence ATG encoding initial Met through AGA encoding the terminal Arg operatively linked to an expression control sequence, and separating the erythropoietin so produced from the cells and the medium.

2. A method of claim 1, wherein the culture medium contains fetal serum.

3. A method of one of the preceding claims, wherein the host cells are mammalian cells.

4. A method of claim 3, wherein the mammalian host cells are COS, CHO, C127 or 3T3 cells.

5. A method of claim 3, wherein the mammalian cells are 3T3 cells.

6. A method of claim 3, wherein the mammalian cells are Chinese hamster ovary (CHO) cells.

7. A method of claim 3, wherein said DNA sequence is contained in a vector also containing bovine papilloma virus DNA.

## Patentansprüche

1. Verfahren zur Herstellung von humanem Erythropoietin, umfassend das Kultivieren von eukaryontischen Wirtszellen in einem geeigneten Medium, die die DNA Sequenz, wie in Tabelle 3 gezeigt, von der Sequenz ATG, kodierend für ein anfängliches Met, bis AGA, kodierend für das terminale Arg enthalten, welche operativ mit einer Expressionskontrollsequenz verknüpft ist, und das Abtrennen des so erzeugten Erythropoietins von den Zellen und dem Medium.

2. Verfahren nach Anspruch 1, worin das Kulturmedium fötales Serum enthält.

3. Verfahren nach einem der vorhergehenden Ansprüche, worin die Wirtszellen Säugerzellen sind.

4. Verfahren nach Anspruch 3, worin die Säuger-Wirtszellen COS, CHO, C127 oder 3T3 Zellen sind.

5. Verfahren nach Anspruch 3, worin die Säugerzellen 3T3 Zellen sind.

6. Verfahren nach Anspruch 3, worin die Säugerzellen Chinesischer-Hamster-Ovarien (CHO) Zellen sind.

7. Verfahren nach Anspruch 3, worin die DNA-Sequenz in einem Vektor enthalten ist, der auch Bovine Papilloma Virus DNA enthält.

## Revendications

1. Procédé de production d'érythropoïétine humaine comprenant la culture dans un milieu approprié de cellules hôtes eucaryotes contenant la séquence d'ADN telle que représentée dans le tableau 3 de la séquence ATG codant la Met initiale à AGA codant la Arg terminale liée de manière active à une séquence de contrôle d'expression, et la séparation de l'érythropoïétine ainsi produite des cellules et du milieu.

2. Procédé selon la revendication 1 dans lequel le milieu de culture contient du sérum foetal.

3. Procédé selon l'une des revendications précédentes dans lequel les cellules hôtes sont des cellules de mammifère.

4. Procédé selon la revendication 3 dans lequel les cellules hôtes de mammifère sont des cellules COS, CHO, C127 ou 3T3.

5. Procédé selon la revendication 3 dans lequel les cellules de mammifère sont des cellules 3T3.

6. Procédé selon la revendication 3 dans lequel les cellules de mammifère sont des cellules d'ovaire de hamster chinois (CHO).

7. Procédé selon la revendication 3 dans lequel ladite séquence d'ADN est contenue dans un vecteur contenant aussi de l'ADN de papillomavirus bovin.
